# EUROPÄISCHE PATENTSCHRIFT

(11) **EP 2 890 810 B1**
(45) Veröffentlichungstag und Bekanntmachung des Hinweises auf die Patenterteilung: **02.08.2017**
(21) Anmeldenummer: 13752832.9
(22) Anmeldetag: 16.08.2013
(51) Int. Cl.: C12Q 1/68

(54) **SENSOR FÜR NADP(H) UND ENTWICKLUNG VON ALKOHOLDEHYDROGENASEN**
SENSOR FOR NADP (H) AND DEVELOPMENT OF ALCOHOL DEHYDROGENASES
CAPTEUR DE NADP(H) ET DÉVELOPPEMENT D'ALCOOL DÉSHYDROGÉNASES

(30) Priorität: 28.08.2012 DE 102012017026
(43) Veröffentlichungstag der Anmeldung: 08.07.2015
(73) Patentinhaber: Forschungszentrum Jülich GmbH, 52428 Jülich (DE)
(72) Erfinder: SIEDLER, Solvej, 52064 Aachen (DE); SCHENDZIELORZ, Georg, 40223 Düsseldorf (DE); BINDER, Stephan, 52249 Eschweiler (DE); EGGELING, Lothar, 52428 Jülich (DE); BRINGER-MEYER, Stephanie, 52428 Jülich (DE); BOTT, Michael, 52428 Jülich (DE)
(74) Vertreter: Herzog, Fiesser & Partner Patentanwälte PartG mbB
(86) Internationale Anmeldenummer: PCT/EP2013/002481
(87) Internationale Veröffentlichungsnummer: WO 2014/032777

(56) Entgegenhaltungen:
- CN-A- 101 386 828
- KRAPP ADRIANA R ET AL: "The soxRS response of Escherichia coli can be induced in the absence of oxidative stress and oxygen by modulation of NADPH content", MICROBIOLOGY (READING), Bd. 157, Nr. Part 4, April 2011 (2011-04), Seiten 957-965, XP002714136, ISSN: 1350-0872
- LEE JIN HYUNG ET AL: "An oxidative stress-specific bacterial cell array chip for toxicity analysis", BIOSENSORS & BIOELECTRONICS, Bd. 22, Nr. 9-10, April 2007 (2007-04), Seiten 2223-2229, XP002714137, ISSN: 0956-5663
- KABIR MD M ET AL: "Investigation into the effect of soxR and soxS genes deletion on the central metabolism of Escherichia coli based on gene expressions and enzyme activities", BIOCHEMICAL ENGINEERING JOURNAL, ELSEVIER, AMSTERDAM, NL, Bd. 30, Nr. 1, 1. Mai 2006 (2006-05-01), Seiten 39-47, XP028034632, ISSN: 1369-703X, DOI: 10.1016/J.BEJ.2006.01.015 [gefunden am 2006-05-01]
- KIM YANG HEE ET AL: "Iron oxide/carbon black (Fe2O3/CB) composite electrode for the detection of reduced nicotinamide cofactors using an amperometric method under a low overpotential", BIOSENSORS & BIOELECTRONICS, Bd. 25, Nr. 5, Januar 2010 (2010-01), Seiten 1160-1165, XP002714138,
- KOBAYASHI KAZUO ET AL: "Activation of SoxR-dependent transcription in Pseudomonas aeruginosa.", JOURNAL OF BIOCHEMISTRY NOV 2004, Bd. 136, Nr. 5, November 2004 (2004-11), Seiten 607-615, XP002714139, ISSN: 0021-924X

## Beschreibung

Die vorliegende Erfindung betrifft eine Zelle, ein Verfahren zum Isolieren von Genen, welche für NADP(H)-abhängige Enzyme kodieren, sowie die Verwendung eines NADP(H)-Nanosensors.

Die Nutzung von NADP(H)-abhängigen Enzymen in der chemischen Industrie als Katalysator ist in einer Vielzahl von Beispielen offenbart. So werden Alkoholdehydrogenasen, auch Oxidoreduktasen oder Ketoreduktasen genannt, zur Reduktion von Carbonylgruppen eingesetzt. Insbesondere wird die Enantiospezifität und Regiospezifität zur Reduktion prochiraler Ketone benutzt. Beispiele solcher Ketoreduktasen die zur Synthese nutzbarer chemischer Verbindungen dienen sind die asymetrische Reduktion von 4-Chloroacetoacetatestern (US 5,559,030, US 5,700,670 und US 5,891,685), die Reduktion von Dicarboxylsäuren (US 6,399,339), die Reduktion von tert-butyl (S) Chloro-5-hydroxy-3-oxohexanoat (US 6,645,746 und WO-A-01/40450), die Reduktion von Pyrrolotriazine-basierten Verbindungen (LJS-A-2006/0286646), die Reduktion substituierter Acetophenone (US 6,800,477, US-A-2012/0178142) oder die Reduktion von Hydroxythiolanen (WO-A-2005/054491). Ebenso werden enzymatisch alpha-halo Ketone zu alpha-halo Alkoholen reduziert. Auch dies kann durch isolierte Enzyme oder mit ganzen Zellen erfolgen (WO-A-2008/038050). Mittels spezifischer Alkoholdehydrogenasen aus *Lactobacillus brevis* oder *Thermoanaerobium brokii* erfolgt die Reduktion des 8-Chloro-6-oxooctanoicsäurealkylesters zum (R) oder (S)-8-Chloro-6-hydroxyoctanoicsäurealkylester, der jeweils als Vorstufe der (R)-α-Liponsäure und (S)-α- Liponsäure Verwendung findet (US 7,157,253). Auch sind Verfahren zur Herstellung von optisch aktiven-Alkanolen beschrieben, wobei durch enzymatische Reduktion der entsprechenden Ketone, die Herstellung von beispielsweise (1S)-3-Methylamino-1-(2-thienyl)-propan-1-ol und (1S)-3-Chlor-1-(2-thienyl)-propan-1-ol erfolgt (WO-A-2006/094945). Ein Verfahren, um mittels Ketoreduktase oder Alkoholdehydrogenase enantiospezifisch 3-Hydroxybutyl-3-hydroxybutyrate herzustellen, ist ebenfalls bekannt (US-A-2012/0064611). In US 6,645,746 wird eine Aminosäuresequenz aus *Candida magnoliae* offenbart, die genutzt werden kann, um mit Hilfe von NADP(H) tert-Butyl (5S)-6-chloro-5-hydroxy-3-oxohexanoat zu tert-Butyl (3R,5S)-6-chloro-3,5-dihydröxyhexanoat zu reduzieren. In der Beschreibung dieses Dokuments wird das Enzym bevorzugt koexprimiert mit Glucosedehydrogenase aus *Bacillus megaterium* eingesetzt, wobei die Regenerierung des Cofaktors NADP(H) mit Hilfe der Glucosedehydrogenase und mit Glucose als Cosubstrat erfolgt. Die WO-A-2004/111083 beschreibt ein Verfahren zur enantioselektiven enzymatischen Reduktion von Ketonen, insbesondere 2- und 3-Oxosäureestern, wobei die Reaktion von einer Oxidoreduktase aus *Pichia capsulata* katalysiert wird. In der WO-A-2005/108593 wird ein Verfahren zur Herstellung von 1-Butanol beschrieben, bei dem 2-Butanon mit einer Carbonylreduktase, beispielsweise aus *Candida parapsilosis,* und einem Coenzym in einem Zweiphasensystem reduziert wird. In der EP-A-2 061 880 ist ein Verfahren zur NADP(H)-abhängigen enzymatischen Herstellung von Alkenon-Derivaten aus a,β-ungesättigten Alkinon-Derivaten offenbart, wobei die entsprechende Reduktase in gereinigter Form oder auch in Form des Mikroorganismus selbst verwendet werden kann. In der EP-A-2 087 127 wird ein Verfahren zur Herstellung von Secolderivaten durch enantioselektive enzymatische Reduktion von Secodionderivaten unter Verwendung einer Oxidoreduktase/Dehydrogenase in Gegenwart von NADP(H) beschrieben.

Neben der NADP(H)-abhängigen Reduktion von Ketonen und Aldehyden werden NADP(H)-abhängige Enzyme, sogenannte Enoatreduktasen, auch zur enantiospezifischen Reduktion von Enoaten benutzt. So berichteten Kataoka und Mitarbeiter, dass durch Nutzung einer Enoatreduktase von *Candida macedoniensis* zusammen mit einer NADP(H) generierenden Glukosedehydrogenase aus *E. coli* präparativ Ketoisophoron zu (6R)-Levodione reduziert wird (Kataoka, Kotaka, Thiwthong, Wada, Nakamori, and Shimizu, J. Biotechnol., 2004, 114, 1-9).

Ferner wird die Nutzung NADP(H)-abhängiger Enzyme in gekoppelten Systemen beschrieben, wo beispielsweise der Reduktion eine Cyclisierung zum Epoxid nachgeschaltet ist. So wird die Nutzung von (R)- oder (S)-selektiven Alkoholdehydrogenasen beschrieben, um das entsprechende Enantiomer zu bilden und anschließend die Basen-induzierte Cyclisierung zum jeweiligen Epoxid zu erreichen (CA 2 612 407).

Die enzymatische Bereitstellung von NADP(H) ist auch erforderlich, wenn Monooxygenasen eingesetzt werden, wie im Falle der sehr gut untersuchten Monooxygenase P450 BM3 (CYP102A1) aus *Bacillus megaterium* (Appl. Microbiol. Biotechnol. (2012) 95:357-367). Diese Fettsäurehydroxylase oxidiert eine breite Palette von Substraten, wie Alkane, Alkene und aromatische Hydrocarbone. Die Monooxygenase katalysiert die Hydroxylierung, sie benötigt aber die stöchiometrische Versorgung mit NADP(H).

Auch werden NADP(H)-abhängige Enzyme zur reduktiven Aminierung eingesetzt, wie beispielsweise von 2-Ketosäuren zur entsprechenden D-Aminosäure (WO-A-2006/113085), oder von 6-Aminocapronsäure aus 2-Ketopimelat (WO-A-2012/031911).

Eine Übersicht über die verschiedensten Anwendungen NADP(H)-abhängiger Enzyme kann zum Beispiel Hollmann, Arendsa und Holtmann (Green Chemistry, 2011, 13, 2285-2313), oder auch dem Lehrbuch "Industrial Biotransformations" von Liese, Seelbach, und Wandrey (Wiley-VCH Verlag, 2006, ISBN: 3-527-31001-0) entnommen werden.

Unabhängig davon, für welche konkrete Umsetzung NADP(H)-abhängige Enzyme eingesetzt werden sollen, ist es zunächst eine Vorraussetzung, geeignete Enzyme zur Verfügung zu stellen, welche hohe Umsätze und eine hohe Stereospezifität gewährleisten. Dieses wiederum setzt ein Screening nach solchen Enzymen voraus, das auf unterschiedliche Weise erfolgen kann.

So bieten Unternehmen Enzymsammlungen an, die dann getestet werden müssen, ob sie das gewünschte Edukt zum gewünschten Produkt umsetzen, wie beispielsweise Novozymes A/S mit Sitz in Bagsvaerd, Dänemark. Gewünschte Enzyme können auch durch Nutzung der natürlichen Diversitivität genutzt werden. Zum Beispiel, indem Enzyme aus Organismen oder Metagenombanken gewonnen werden, die wiederum spezifisch getestet werden müssen. Die Diversitivität kann auch durch den Menschen hergestellt werden, indem vorhandene Enzyme mutagenisiert werden, und anschließend gewonnene Enzyme auf veränderte Substratspezifität getestet werden. Beispiele zur Erzeugung diverser Enzyme durch molekulare Techniken sind in der WO-A-2012/069434 offenbart, wo NADP(H)-abhängige Enzyme zur Herstellung n-heterozyklischer optisch aktiver Alkohole gewonnen werden. Auch sind ähnliche Verfahren zur Herstellung von 12 α-Hydroxysteroiddehydrogenase-Mutanten beschrieben (EP-A-2 441 771). Die Herstellung großer Genbanken, die einer Analyse mit hohem Durchsatz unterliegen, umfasst das Klonieren der Genbank in replizierbare Expressionsvektoren, das Transformieren der geeigneten Zellen mit der resultierenden Vektorenbank und das Exprimieren der kombinatorisch erhaltenen Gene unter Bedingungen, unter denen der Nachweis der gewünschten Aktivität und die Isolation des Vektors, der das Gen kodiert, dessen Produkt nachgewiesen wurde, erleichtert.

Der unmittelbare Test auf gewünschte Umwandlung des Edukts zum Produkt erfolgt bisher bevorzugt in Mikrotiterplatten mit 96, 384 oder auch 1536 Vertiefungen ("*wells*")*.* Diese Platten ermöglichen den parallelen Test von 96, 384, oder 1536 Enzymen. Das Produkt der gewünschten Enzymreaktion kann direkt durch chromatographische Techniken bestimmt werden. Diese Methode erfordert die Entnahme einer Probe aus den 96, 384 oder 1536 wells und die chromatographische Trennung zum Nachweis der Reaktionsprodukte, bei denen es sich beispielsweise um Alkohole oder Carbonylverbindungen handeln kann. Solch eine Prozedur ist allerdings komplex und zeitaufwendig. Deswegen werden oft indirekte Tests angewendet. So wird ausgenutzt, dass NADP(H) bei 340 nm absorbiert, nicht aber NADP. Darüber kann prinzipiell die verbrauchte NADP(H)-Menge bestimmt werden. Alternativ kann so auch bei der Carbonylreduktasekatalysierten Oxidation eines Alkohols die Umwandlung von NADP zu NADP(H) gemessen werden. In dieser und vergleichbaren Reaktionen wird die Reduktion des Co-Faktors NADP durch Zunahme der Absorbtion bei 340 nm bestimmt. Gleichermaßen kann auch die Eigenfluoreszenz des reduzierten Cofaktors zur Quantifizierung benutzt werden. Dies geschieht in Mikrotiterauslesegeräten.

In einem anderen Verfahren zur Bestimmung des NADP(H)-Verbrauchs zum Nachweis der enzymatischen reduktiven Transaminierung und auch der Reduktion von Ketonen wird die mit dem NADP(H)-Verbrauch einhergehende pH-Änderung durch einen Farbindikator bestimmt (US 7,642,073). Durch eine geeignete Wahl des Farbindikators kann die Wellenlänge der Farbänderung bestimmt werden, die wiederum in Mikrotiterauslesegeräten bestimmt wird.

Auch sind spezielle Mikrotiterplatten-Systeme beschrieben, in denen über Membranen mit spezifischen Analyt-bindenden Eigenschaften und Flüssigkeitsströmen ein Screening im Mikrotiterplattenformat mit bis zu 1536 wells durchgeführt wird (EP-A-1 628 768).

Auch ist versucht worden, Analyten durch Kopplung mit einer nachweisbaren Gruppe, beispielsweise eines Fluorophors, leichter nachweisbar zu machen. Dazu wird der Analyt vor Durchführen der Reaktion kovalent mit einer fluoreszierenden Gruppe verbunden. Bei Durchführen der Reaktion und entsprechender Umsetzung des Analyten soll die Fluoreszenz der fluoreszierenden Gruppe geändert werden, beispielsweise durch Abspaltung der Gruppe oder durch Änderung der Struktur des Analyten. Die Fluoreszenzänderung ist dann ein Maß für die Umsetzung des Analyten. Nachteilig hieran ist jedoch, dass die fluoreszierende Gruppe die Reaktionsfähigkeit des Analyten häufig beeinflusst. In der WO-A-2007/131696 wird beschrieben, dass durch Bereitstellen eines Fluoreszenzfarbstoffs und eines Makrozyklus in der zu untersuchenden Probe und Messen einer Fluoreszenzeigenschaft des Fluoreszenzfarbstoffs an zumindest zwei Zeitpunkten die Analytkonzentration bestimmt werden kann. Dabei bindet der Makrozyklus den Farbstoff und innerhalb des zu untersuchenden Konzentrationsbereiches des Analyten verdrängt dieser den Fluoreszenzfarbstoff vom Makrozyklus.

Bei den aus dem Stand der Technik bekannten *in vitro* Screening-Ansätzen zum Isolieren neuer NADP(H)-verbrauchender Enzyme oder NADP(H)-verbrauchender Enzyme aus Genbanken mit veränderter Substratspezifität ist ein genereller Nachteil, dass Microtiterplatten-Systeme benutzt werden, die kein Hochdurchsatzscreening ermöglichen, wie es zum Beispiel mit Fluoreszenz aktivierter Zellsortierung (FACS, Fluorescence Activated Cell Sorting) möglich ist.

Darüberhinaus werden in *in vitro* Screening-Ansätzen zum Isolieren neuer NADP(H)-abhängiger Enzyme oft Zelllysate als potentielle Quelle neuer Enzyme eingesetzt, da die Isolierung in reiner Form operationell schwierig ist. Das Problem solcher Lysate oder Präparate beim Routinescreening nach neuen NADP(H)-abhängigen Enzymen besteht jedoch darin, dass der Reaktionsansatz typischerweise nicht lösliches Material oder andere Enzyme enthält, die mit dem NADP(H) interagieren. Dies führt zu hohen Blindwerten oder auch veränderter unspezifischer Absorbtion bei 340 nm, was die Genauigkeit und den Wert der Absobtionsmessung schmälert. Das gleiche gilt für Fluoreszenzmessung des Cofaktors, die ebenfalls durch unlösliches Material erschwert wird.

Adriana R. Krapp et al. ("The SoxRS response of Escherichia coli can be induced in the absence of oxidative stress and oxygen by modulation of NADPH contents", Microbiology 157(4), Seiten 957-965 (2011)) befassen sich mit dem soxRS-Regulon in E.coli. Es wird festgestellt, dass die Aktivierung des soxRS-Regulons in E. coli-Zellen, die Verbindungen ausgesetzt sind, welche die Bildung von Superoxid fördern, eher durch den Abbau des NADPH-Pools als durch die Anreicherung von Superoxid selbst ausgelöst wird.

Jin Hyung Lee et al. ("An oxidative stress-specific bacterial cell array chip for toxicity analysis", Biosens Bioelectron. 22(9-10), Seiten 2223-2229 (2006)) beschreiben einen für oxidativen Stress spezifischen bakteriellen Array-Chip, der zur Toxizitäts-Analyse eingesetzt wird.

Yang Hee Kim et al. ("Iron oxide/carbon black (Fe203/CB) composite electrode for the detection of reduced nicotinamide cofactors using an amperometric method under a low overpotential", Biosens Bioelectron. 25(5), Seiten 1160-1165 (2009)) beschreiben einen amperometrischen Biosensor zur Detektion der Co-Faktoren NADH und NADPH basierend auf der elektrochemischen Oxidation von NADPH mit einer Eisenoxid /CB-Elektrode.

CN 101386828 B offenbart einen *E. coli*-Stamm, der erhalten worden ist, im dem im *E. coli*-Stamm MC4100 die Gene *sodA, sodB*, *katG, ahpCF* und *frdABCD* ausgeschaltet und das *soxS*-Gen sowie das *gfp*-Reportergen mittels rekombinanter Verfahren eingeführt worden sind. Die Zellen dienen zum Nachweis von redoxaktiven Toxinen.

Der vorliegenden Erfindung lag die Aufgabe zugrunde, die sich aus dem Stand der Technik ergebenden Nachteile im Zusammenhang mit der Isolierung neuer NADP(H)-abhängiger Enzyme zu überwinden.

Insbesondere lag der vorliegenden Erfindung die Aufgabe zugrunde, ein Werkzeug zur Verfügungs zu stellen, welches verwendet werden kann, um in einem Hochdurchsatzscreening, beispielsweise mittels FACS, aus einer Zellsupension in möglichst einfacher Art und Weise diejenigen Zellen isolieren zu können, welche gegebenenfalls neue NADP(H)-abhängige Enzyme exprimieren. Insbesondere soll das Isolieren dieser Zellen keinen Zellaufschluss und insbesondere auch keine analytische Bestimmung der Konzentration von bestimmten Edukten, Produkten oder Co-Faktoren umfassen.

Darüberhinaus lag der vorliegenden Erfindung die Aufgabe zugrunde, eine Zelle bereitzustellen, die, nachdem ein Gen für ein potentielles NADP(H)-abhängiges Enzym beispielsweise in Form eines Plasmids in die Zelle eingebracht worden ist, besonders leicht und insbesondere ohne die Notwendigkeit eines Zellaufschlusses dahingehend analysiert werden kann, ob das von dieser Zelle exprimierte Gen in der Tat für ein NADP(H)-abhängiges Enzym kodiert. Eine auf diese Art und Weise identifizierte Zelle sollte sich darüberhinaus möglichst in einem Hochdurchsatzscreening, beispielsweise mittels FACS, aus einer Vielzahl von Zellen, beispielsweise aus einer Zellsuspension, gezielt abtrennen lassen.

Einen Beitrag zur Lösung der eingangs genannten Aufgaben leistet eine Zelle beinhaltend einen NADP(H)-Nanosensor, wobei der NADP(H)-Nanosensor
i) eine Nukleinsäuresequenz, an die ein Regulator zu binden vermag, wobei der Oxidationszustand des Regulators von der NADP(H)-Verfügbarkeit abhängig ist;
ii) eine sich an die Nukleinsäuresequenz i) anschließende Promotorsequenz, an die eine RNA-Polymerase zu binden vermag, wobei die Affinität der RNA-Polymerase für die Promotorsequenz durch den Oxidationszustand des Regulators beinflusst wird; promotors
iii) eine sich unter der Kontrolle der Promotorsequenz ii) befindliche Nukleinsäuresequenz, die für ein Autofluoreszenzprotein kodiert umfasst, wobei die Nukleinsäuresequenz i) die SoxR-Bindesequenz, der Regulator der Sox-Regulator (SoxR) und die Promotorsequenz die soxS-Promotorsequenz ist und wobei die Zelle weiterhin ein Plasmid mit einem gegebenenfalls mutierten Gen, welches für ein NADP(H)-abhängiges Enzym kodiert, umfasst.

Überraschend wurde festgestellt, dass sich unter Verwendung des NADP(H)-Nanosensors *in vivo* die intrazelluläre NADP- bzw. NADP(H)-Konzentration und damit indirekt die Aktivität NADP(H)-abhängiger Enzyme in einer Zelle besonders leicht bestimmen lässt. Ist eine den NADP(H)-Nanosensor aufweisende Zelle durch eine hohe Aktivität NAPD(H)-abhängiger Enzyme gekennzeichnet, so ist die Konzentration an NADP entsprechend hoch (und die NADP(H)-Konzentration entsprechende gering). In Abhängigkeit dieses Reduktionszustandes der Zelle vermag der Regulator die Affinität der RNA-Polymerase für den die Expression des Autofluoreszenzsproteins steuernden Promotor oder die Stabilität der für das Autofluoreszenzprotein kodierenden mRNA zu beeinflussen. In Abhägigkeit vom Reduktionszustand der Zelle wird somit die Expression des Autofluoreszensproteins gesteuert, die wiederum in einfacher Weise durch das Einstrahlen mit elektromagnetischer Strahlung, welche das Autofluoreszenzprotein zur Emission von Licht anregt, verfolgt werden kann. Die Lichtemission der Zellen ist somit ein Indikator für den Reduktionszustand der Zelle und mithin für das Ausmaß der Expression NADP(H)-abhängiger Enzyme.

Erfindungsgemäß handelt es sich bei dem Regulator um den Sox-Regulator (SoxR) und bei der Promotorsequenz um die *soxS*-Promotorsequenz. Das Gen für SoxR aus *E. coli* K12 ist unter den Zugangsnummern b4063, ECK4055 im National Center for Biotechnology Information (NCBI) database of the National Library of Medicine (Bethesda, MD, USA) hinterlegt. SoxR enthält zwei [2Fe-2S]-Cluster, die essentiell für die Transkriptionsaktivität sind. Jedes SoxR-Polypeptid enthält einen [2Fe-2S]-Cluster, der den Reduktionszustand der Zelle detektiert. Sowohl Fe-SoxR als auch apo-SoxR binden an die Promoter-Region, aber nur Fe-SoxR trägt zur Promoteraktivierung in der oxidierten Form bei. Der Redoxzustand des Eisen-Schwefel Clusters reguliert die SoxR-Aktivität. Das Zielgen von SoxR ist das benachbarte *soxS,* dessen Sequenz unter den Nummern b4062, ECK4054 im National Center for Biotechnology Information (NCBI) database of the National Library of Medicine (Bethesda, MD, USA) hinterlegt ist. Der Reduktionszustand der Zelle kann gegebenfalls durch NADP(H)-abhängige Reduktasen, wie Rsx oder RseC, begünstigt werden.

In diesem Zusammenhang ist es weiterhin bevorzugt, dass die Komponenten i) und ii) durch den intergenischen Bereich aus *E. coli,* der zwischen *soxR* und *soxS* lokalisiert ist und der die SoxR-Bindesequenz, die sich an die SoxR-Bindesequenz anschließende *soxS-*Promotorsequenz und eine sich an die soxS-Promotorsequenz anschließende Sequenz, die auf der Ebene der mRNA einer Ribosomenbindesstelle entspricht, umfasst, oder durch eine hierzu homologe Nukleinsäuresequenz gebildet werden. Die Komponenten i) und ii) werden dabei vorzugsweise gebildet von einer Nukleinsäuresequenz ausgewählt aus der Gruppe bestehend aus:
a) einer Nukleinsäuresequenz gemäß SEQ.-ID-Nr. 01,
b) einer Nukleinsäuresequenz, die eine Identität von mindestens 70 %, vorzugsweie mindestens 80 %, noch mehr bevorzugt mindestens 85 %, noch mehr bevorzugt mindestens 90 %, noch mehr bevorzugt mindestens 91 %, noch mehr bevorzugt mindestens 92 %, noch mehr bevorzugt mindestens 93 %, noch mehr bevorzugt mindestens 94 %, noch mehr bevorzugt mindestens 95 %, noch mehr bevorzugt mindestens 96 %, noch mehr bevorzugt mindestens 97 %, noch mehr bevorzugt mindestens 98 % und am meisten bevorzugt mindestens 99 % zur Nukleinsäuresequenz von a) aufweist, wobei die Nukleinsäuresequenz in der Lage ist, SoxR dergestalt zu binden, dass die Affinität der RNA-Polymerase für den *soxS-*Promotor vom Oxidationszustand von SoxR abhängig ist, und
c) einer Nukleinsäuresequenz, die unter stringenten Bedingungen mit einer komplementären Nukleinsäuresequenz nach a) oder b) zu hybridisieren vermag, wobei die Nukleinsäuresequenz in der Lage ist, SoxR dergestalt zu binden, dass die Affinität der RNA-Polymerase für den *soxS*-Promotor vom Oxidationszustand von SoxR abhängig ist.

Gemäß einer ersten Variante dieser besonders bevorzugten Ausführungsform des NADP(H)-Nanosensors umfasst dieser
(α1) das *E. coli-*Gen für SoxR (*soxR*) oder eine hierzu homologe Nukleinsäuresequenz;
(α2) den sich an (α1) anschließenden intergenischen Bereich aus *E. coli,* der zwischen *soxR* und *soxS* lokalisiert ist und der die SoxR-Bindesequenz, die sich an die SoxR-Bindesequenz anschließende *soxS*-Promotorsequenz und eine sich an die soxS-Promotorsequenz anschließende Sequenz, die auf der Ebene der inRNA einer Ribosomenbindestelle entspricht, umfasst, oder eine hierzu homologe Nukleinsäuresequenz, wie vorstehend definiert, als Komponenten i) und ii);
(α3) gegebenenfalls eine sich an (α2) anschließende Teilsequenz des *soxS*-Gens aus *E. coli* oder eine hierzu homologe Nukleinsäuresequenz;
(α4) eine sich an (α2) oder (α3), vorzugsweise an (α3) anschließende und unter der Kontrolle der *soxS*-Promotorsequenz befindliche Nukleinsäuresequenz, welche für ein. Autofluoreszenzprotein kodiert, als Komponente iii).

Die Formulierung *"eine sich an eine Sequenz a) anschließende Sequenz b)",* wie sie vorstehend und auch nachfolgend verwendet wird, ist erfindungsgemäß so zu verstehen, dass die Sequenz b) nicht zwingend unmittelbar mit der Sequenz a) verbunden sein muss, sondern dass auch eine Zwischensequenz zwischen der Sequenz a) und der Sequenz b) lokalisiert sein kann.

Als Komponente (α1) umfasst der NADP(H)-Nanosensor gemäß dieser besonderen Ausgestaltung das *E. coli*-Gen für SoxR (*soxR*) oder eine hierzu homologe Nukleinsäuresequenz, wobei die Komponente (α1) vorzugsweise ausgwählt ist aus der Gruppe bestehend aus:
a) einer Nukleinsäuresequenz gemäß SEQ.-ID.-Nr. 02,
b) einer Nukleinsäuresequenz, kodierend ein Polypeptid mit einer Aminosäuresequenz gemäß SEQ.-ID.-Nr. 03,
c) einer Nukleinsäuresequenz, die eine Identität von mindestens 70 %, vorzugsweie mindestens 80 %, noch mehr bevorzugt mindestens 85 %, noch mehr bevorzugt mindestens 90 %, noch mehr bevorzugt mindestens 91 %, noch mehr bevorzugt mindestens 92 %, noch mehr bevorzugt mindestens 93 %, noch mehr bevorzugt mindestens 94 %, noch mehr bevorzugt mindestens 95 %, noch mehr bevorzugt mindestens 96 %, noch mehr bevorzugt mindestens 97 %, noch mehr bevorzugt mindestens 98 % und am meisten bevorzugt mindestens 99 % zur Nukleinsäuresequenz von a) oder b) aufweist, wobei die Nukleinsäuresequenz ein Polypeptid kodiert, welches an die SoxR-Bindesequenz im intergenischen Bereich aus *E. coli,* der zwischen *soxR* und *soxS* lokalisiert ist, zu binden vermag und dessen Oxidationszustand die Affinität der RNA-Polymerase für die ebenfalls im intergenischen Bereich aus *E. coli* lokalisierte Promotorsequenz zu beeinflussen vermag,
d) einer Nukleinsäuresequenz, kodierend ein Polypeptid, das eine Homologie von mindestens 70 %, vorzugsweise mindestens 80 %, noch mehr bevorzugt mindestens 85 %, noch mehr bevorzugt mindestens 90 %, noch mehr bevorzugt mindestens 91 %, noch mehr bevorzugt mindestens 92 %, noch mehr bevorzugt mindestens 93 %, noch mehr bevorzugt mindestens 94 %, noch mehr bevorzugt mindestens 95 %, noch mehr bevorzugt mindestens 96 %, noch mehr bevorzugt mindestens 97 %, noch mehr bevorzugt mindestens 98 % und am meisten bevorzugt mindestens 99 % zur SEQ.-ID.-Nr. 03 aufweist, wobei die Nukleinsäuresequenz ein Polypeptid kodiert, welches an die SoxR-Bindesequenz im intergenischen Bereich aus *E. coli,* der zwischen *soxR* und *soxS* lokalisiert ist, zu binden vermag und dessen Oxidationszustand die Affinität der RNA-Polymerase für die ebenfalls im intergenischen Bereich aus *E. coli* lokalisierte Promotorsequenz zu beeinflussen vermag, und
e) einer Nukleinsäuresequenz, die unter stringenten Bedingungen mit einer komplenetären Nukleinsäuresequenz nach einer der Gruppen a) bis d) zu hybridisieren vermag, wobei die Nukleinsäuresequenz ein Polypeptid kodiert, welches an die SoxR-Bindesequenz im intergenischen Bereich aus *E. coli,* der zwischen *soxR* und *soxS* lokalisiert ist, zu binden vermag und dessen Oxidationszustand die Affinität der RNA-Polymerase für die ebenfalls im intergenischen Bereich aus *E. coli* lokalisierte Promotorsequenz zu beeinflussen vermag.

Der Ausdruck "Homologie" (oder "Identität"), wie hierin verwendet, kann durch die Gleichung H (%) = [1-V/X]x 100, definiert werden, worin H Homologie bedeutet, X die Gesamtzahl an Nukleobasen/Aminosäuren der Vergleichssequenz ist und V die Anzahl an unterschiedlichen Nukleobasen/Aminosäuren der zu betrachtenden Sequenz bezogen auf die Vergleichssequenz ist. Auf jeden Fall sind mit dem Begriff Nukleinsäuresequenzen, welche für Polypeptide kodieren, alle Sequenzen umfasst, die nach Maßgabe der Degeneration des genetischen Codes möglich erscheinen.

Die Identität von Nukleinsäuresequenzen läßt sich unter Anwendung eines Sequenz-Vergleichs-Programms (BLAST, Altschul et al. J. Mol. Biol. 1990, 215, 403-410) identifizieren. Die prozentuale Homologie zwischen zwei Aminosäuresequenzen kann ebenfalls mit aus dem Stand der Technik bekannten Verfahren vom Fachmann ohne weiteres ermittelt werden. Ein geeignetes Programm, das erfindungsgemäß eingesetzt werden kann, ist BLASTp (Altschul et al.. 1997; "Gapped BLAST and PSI-BLAST: a new generation of protein database search programs"; Nucleic Acids Res. 25(17): 3389-3402).

Anleitungen zur Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde, beispielsweise die zu *soxR* oder *soxS* oder dem intergenischen Bereich von *soxRS* aus *E. coli* komplementäre Nukleotidsequenz, und Zielsequenz, d. h. die mit der Sonde behandelten Polynukleotide, mindestens 70 % identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5 × SSC-Puffer bei einer Temperatur von ca. 50°C - 68°C eingesetzt werden. Dabei können Sonden auch mit Polynukleotiden hybridisieren, die weniger als 70 % Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2 × SSC und gegebenenfalls nachfolgend 0,5 × SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C. eingestellt wird. Vorzugsweise werden die Waschschritte bei Temperaturen von ca. 62°C - 68°C, bevorzugt von 64°C - 68°C oder ca. 66°C - 68°C, besonders bevorzugt von ca. 66°C - 68°C durchgeführt. Es ist gegebenenfalls möglich die Salzkonzentration bis auf eine Konzentration entsprechend 0,2 × SSC oder 0,1 × SSC zu senken. Durch schrittweise Erhöhung der Hybridisierungstemperatur in Schritten von ca. 1 - 2°C von 50°C bis 68°C können Polynukleotidfragmente, die für *soxR* oder *soxS* oder dem intergenischen Bereich von *soxRS* kodieren, isoliert werden, die beispielsweise mindestens 70 % oder mindestens 80 % oder mindestens 90 % bis 95 % oder mindestens 96 % bis 98 % oder mindestens 99 % Identität zur Sequenz der eingesetzten Sonde besitzen. Weitere Anleitungen zur Hybridisierung sind in Form sogenannter Kits am Markt erhältlich (z. B. DIG Easy Hyb von der Firma Roche Diagnostics GmbH, Mannheim, Deutschland, Catalog No. 1603558).

Als Komponente (α4) umfasst der NADP(H)-Nanosensor gemäß dieser besonderen Ausgestaltung eine sich an (α2) oder (α3), vorzugsweise an das Zielgen *soxS* (α3), insbesondere an die ersten 5 bis 200 Nukleotide des Zielgens *soxS* anschließende und unter der Kontrolle der *soxS*-Promotorsequenz befindliche Nukleinsäuresequenz, welche für ein Autofluoreszenzprotein kodiert, als Komponente iii).

Die für das Autofluoreszenzprotein kodierende Gensequenz gemäß der Komponente iii) befindet sich erfindungsgemäß unter der Kontrolle der Promotorsequenz ii) (gemäß der vorstehend beschriebenen ersten Variante der besonderen Ausführungsform des NADP(H)-Nanosensor befindet sich die für das Autofluoreszenzprotein kodierende Gensequenz (α4) unter der Kontrolle der *soxS*-Promotorsequenz). Der Begriff *"unter der Kontrolle der Promotorsequenz"* ist dabei vorzugsweise so zu verstehen, dass die für das Autofluoreszenzprotein kodierende Gensequenz mit dem Promotor funktionell verknüpft ist. Funktionell verknüpft sind der Promotor und die für das Autofluoreszenzprotein kodierende Gensequenz, wenn diese beiden Sequenzen sowie gegebenenfalls weitere regulative Elemente, wie zum Beispiel ein Terminator oder eine Ribosomenbindestelle, derart sequentiell angeordnet sind, dass jedes der regulativen Elemente seine Funktion bei der transgenen Expression der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die für das Autofluoreszenzprotein kodierende Gensequenz hinter (d. h. am 3'-Ende) der Promotorsequenz positioniert wird, so dass beide Sequenzen kovalent miteinander verbunden sind. Bevorzugt ist dabei der Abstand zwischen der für das Autofluoreszenzprotein kodierenden Gensequenz und der Promotorsequenz kleiner als 200 Basenpaare, besonders bevorzugt kleiner als 100 Basenpaare, ganz besonders bevorzugt kleiner als 50 Basenpaare. Auch ist es möglich, dass die für das Autofluoreszenzprotein kodierende Gensequenz und der Promotor derart funktionell miteinander verknüpft sind, dass zwischen diesen beiden Gensequenzen noch eine Teilsequenz des homologen Gens (also desjenigen Gens, dessen Expression in der Wildtyp-Zelle durch den Promotor reguliert wird) befinden (gemäß der vorstehend beschriebenen besonderen Ausführungsform des NADP(H)-Nanosensor können sich demnach Teile des *soxS*-Gens gemäß der Komponente (α3) zwischen der soxS-Promotorsequenz und der für das Autofluoreszenzprotein kodierende Nukleinsäuresequenz (α4) befinden). Bei der Expression eines solchen DNA-Konstruktes wird ein Fusionsprotein aus dem Autofluoreszenzprotein und der Aminosäuresequenz, welche von der entsprechenden Teilsequenz des homologen Gens kodiert wird, erhalten (= translationale Fusion). Die Länge solcher Teilsequenzen des homologen Gens sind unkritisch, solange die Funktionsfähigkeit des Autofluoreszenzproteins, also seine Eigenschaft, bei Anregung mit Licht einer bestimmten Wellenlänge zu fluoreszieren, nicht nennenswert beeinträchtigt wird. Im Falle der vorstehend beschriebenen besonderen Ausführungsform des NADP(H)-Nanosensors umfasst die soxS-Teilsequenz (α3) vorzugsweise mindestens die ersten 5 Nukleotide, noch mehr bevorzugt mindestens die ersten 10 Nukleotide und noch mehr bevorzugt mindestens die erstens 20 Nukleotide, vorzugsweise jedoch höchstens die ersten 200 Nukleotide, noch mehr bevorzugt höchstens die ersten 150 Nukleotide und noch mehr bevorzugt höchstens die ersten 100 Nukleotide des *soxS*-Gens.

Die für ein Autofluoreszenzprotein kodierende Nukleinsäuresequenz (iii) (bzw. (α4) und (β4)) umfasst bevorzugt Gene kodierend für Fluoreszenzproteine, welche für fluoreszierende Proteine der Gattung *Aequora* kodieren, wie das Green Fluorescent Protein (GFP), und Varianten davon, die in einem anderen Wellenlängenbereich fluoreszieren (z. B. Yellow Fluorescent Protein (YFP), Blue Fluorescent Protein (BFP), Cyan Fluorescent Protein (CFP)) oder deren Fluoreszenz verstärkt ist (z. B. enhanced Green Fluorescent Protein (EGFP), enhanced Yellow Fluorescent Protein (EYFP), enhanced Blue Fluorescent Protein (EBFP) oder enhanced Cyan Fluorescent Protein (ECFP). Ferner können erfindungsgemäß auch Gensequenzen verwendet werden, welche für andere autofluoreszierende Proteine, z. B. DsRed, HcRed, AsRed, AmCyan, ZsGreen, AcGFP, ZsYellow, wie sie von BD Biosciences, Franclin Lakes, USA, bekannt sind, kodieren. Ebenso kann ein Photoreceptorprotein benutzt werden, das eine sogenannte LOV Domäne enthält. Das besonders bevorzugte Autofluoreszenzprotein ist dabei EYFP.

Gemäß einer zweiten Variante der besonders bevorzugten Ausführungsform des NADP(H)-Nanosensors umfasst dieser
(β1) das *E. coli*-Gen für SoxR (*soxR*) oder eine hierzu homologe Nukleinsäuresequenz;
(β2) den sich an (β1) anschließenden intergenischen Bereich aus *E. coli,* der zwischen *soxR* und *soxS* lokalisiert ist und der die SoxR-Bindesequenz, die sich an die SoxR-Bindesequenz anschließende soxS-Promotorsequenz und eine sich an die soxS-Promotorsequenz anschließende Sequenz, die auf der Ebene der mRNA einer Ribosomenbindestelle entspricht umfasst, oder eine hierzu homologe Nukleinsäuresequenz, wie vorstehend definiert, als Komponenten i) und ii);
(β3) die sich an (β2) anschließende und unter der Kontrolle der soxS-Promotorsequenz befindliche Sequenz des *soxS-*Gens aus *E. coli,* eine Teilsequenz dieses Gens oder einer hierzu homologe Nukleinsäuresequenz;
(β3') eine weitere, sich an (β3) anschließende Sequenz, die auf mRNA-Ebene einer Ribosomenbindestelle entspricht;
(β4) eine sich an (β3') anschließende und unter der Kontrolle der *soxS-*Promotorsequenz befindliche Nukleinsäuresequenz, welche für ein Autofluoreszenzprotein kodiert, als Komponente iii).

Als Komponenten (β1), (β2), (β3) und (β4) sind diejenigen Komponenten bevorzugt, die bereits vorstehend im Zusammenhang mit der ersten Variante der besonders bevorzugten Ausführungsform des NADP(H)-Nanosensors als bevorzugte Komponenten (α1), (α2), (α3) und (α4) genannt wurden. Bei der Expression eines solchen DNA-Konstruktes wird SoxS oder ein Fragment dieses Proteins und, getrennt davon, das Autofluoreszenzprotein gebildet (= transkriptionelle Fusion).

Als Beispiele geeigneter Zellen seien insbesondere Escherichia coli, Pseudomonas fluorescens, Corynebacterium glutamicum, Bacillus subtilis oder ein anderes Eubakterium, oder auch Saccharomyces cerevisiae oder eine andere Hefe genannt.

Die erfindungsgemäßen Zellen eignen sich, um festzustellen, ob bestimmte Gensequenzen für ein NADP(H)-abhängiges Enzym kodieren. Dazu wird das für ein potentielles NADP(H)-abhängiges Enzym kodierende Gen in die Zelle eingebracht und exprimiert. Wie eingangs beschrieben, ist die Lichtemission der Zellen ein Indikator für den Reduktionszustand der Zelle und mithin für das Ausmaß der Expression NADP(H)-abhängiger Enzyme.

Unter einem "NADP(H)-abhängigen Enzym" wird dabei erfindungsgemäß jedes Enzym verstanden, welches bei zumindest einem Teilschritt der Umsetzung eines Substrates in ein von diesem Substrat chemisch verschiedendes Reaktionsprodukt beteiligt ist, wobei in zumindest einem Teilschritt dieser Umsetzung NADP(H) als Co-Faktor beteiligt ist.

Die erfindungsgemäße Zelle umfasst demnach neben dem NADP(H)-Nanosensor weiterhin ein Plasmid mit einem gegebenenfalls mutierten Gen, welches für ein NADP(H)-abhängiges Enzym kodiert. Das NADP(H)-abhängige Enzym ist dabei vorzugsweise ausgewählt aus der Gruppe bestehend aus Alkoholdehydrogenasen, Aldehyddehydrogenasen, Laktatdehydrogenasen, Enoatreduktasen Epoxidreduktasen, Diaminopimelatdehydrogenasen, Aminosäuredehydrogenasen, Aldehydoxidoreduktasen, Alkanreduktasen, Aminreduktasen, Epoxiddehydrogenasen, Carboxylsäuredehydrogenasen, Hydroxysäureketoreduktasen und Hydroxysäuredehalogenasen.

Einen Beitrag zur Lösung der eingangs genannten Aufgaben leistet auch ein Verfahren zum Isolieren von Genen, welche für NADP(H)-abhängige Enzyme kodieren, umfassend die Verfahrensschritte:
(I) Bereitstellung eines NADP(H)-Nanosensors, wie vorstehend im Zusammenhang mit der erfindungsgemäßen Zelle beschrieben;
(II) Einbringen des NADP(H)-Nanosensors in eine Zelle;
(III) Einbringen eines Gens, welches gegebenenfalls für ein NADP(H)-abhängiges Enzym kodiert, in einzelne Zellen einer Zellsuspension aus den im Verfahrensschritt (II) erhaltenen Zellen;
(IV) Inkubieren der Zellen mit einem Substrat für das NADP(H)-anhängige Enzym;
(V) Identifizieren einzelner Zellen in der Zellsuspension mit erhöhter Aktivität NADP(H)-abhängiger Enzyme durch Detektion der intrazellulären Fluoreszenzaktivität;
(VI) Abtrennen der identifizierten Zellen aus der Zellsuspension;
(VII) Isolierung der für ein NADP(H)-abhängiges Enzym kodierenden Gene in den identifizierten Zellen.

Mit Hilfe dieses Verfahrens können neue NADP(H)-abhängige Enzyme und mutierte NADP(H)-abhängige Enzyme mit erhöhter oder veränderter Substraterkennung isoliert werden.

Als NADP(H)-Sensor und als Zelle sind diejenigen Sensoren bzw. Zellen bevorzugt, die bereits eingangs im Zusammenhang mit der erfindungsgemäßen Zelle als bevorzugte Sensoren bzw. Zellen beschrieben wurden.

In den Verfahrensschritten I) und II) wird zunächst eine erfindungsgemäße Zelle hergestellt, indem der NADP(H)-Nanosensor in eine Zelle eingebracht wird, wobei dieses Einbringen in episomaler oder chromosomaler Form erfolgen kann.

Im Verfahrensschritt III) des erfindungsgemäßen Verfahrens wird sodann ein Gen, welches gegebenenfalls für ein NADP(H)-abhängiges Enzym kodiert, in einzelne Zellen einer Zellsuspension aus den im Verfahrensschritt (II) erhaltenen Zellen eingebracht, wobei es sich bei dem Gen insbesondere um ein mutiertes, plasmidkodiertes Gen eines NADP(H)-abhängigen Enzyms handeln kann. Zur Einführung der ortsunspezifischen Mutationen in die plasmidkodierten Gene der NADP(H)-abhängigen Enzyme zur Erhöhung der Diversität wird bevorzugt eine in vitro Mutagenese unter zuhilfenahme einer fehlerhaften Polymeraseketenreaktion (PCR) und einer Amplifikationstechnik durchgeführt. Dabei wird das zu mutierende Gen einer PCR unter Verwendung einer Polymerase unterzogen, die abhängig von den Bedingungen der Reaktion einzelne Basen falsch in die synthetisierten Gene einbaut (Tindall, K.R. and T.A. Kunkel: "Fidelity of DNA synthesis by the Thermus aquaticus DNA polymerase"; Biochemistry, 1988. 27 (16), Seiten 6008-13). Eine häufige Variante dieser Methode beinhaltet die Verwendung von Mangan(II)-Ionen oder von NukJeotidanaloga im PCR Ansatz (Cadwell R. C et al. (1992); PCR Methods Appl. (2), Seiten 28-33./Leung D. W. et al. (1989) Techniques (1), Seiten 11-15). Diese Techniken zur Mutationseinführung werden als "Error-Prone-PCR (epPCR)" bezeichnet (Labrou NE: "Random mutagenesis methods for in vitro directed enzyme evolution"; Curr Protein Pept Sci. 2010 (11), Seiten :91-100). Die Mutationen können beispielsweise Punktmutationen sein, es können z.B. Substitutionen, Deletionen oder Insertionen durch die Polymerase erzeugt werden. Die Mutationsrate beträgt zwischen 1-40 Mutationen pro 1 kb, bevorzugt 1-5 Mutationen pro 1 kb. Mutationen können aber auch mit Hilfe der Sättigungsmutagenese unter Verwendung des Stratagene QuikChange Kit (La Jolla, Kalifornien, USA) hergestellt werden, oder auch mit einer als Sesam bezeichneten Methode (EP 1670914 B1), mit der jedes vorhandene Nukleotid sättigend in jedes mögliche Nukleotid überführt wird.

Als NADP(H)-abhängige Enzyme, deren Aktivität sich mit dem Nanosensortragenden Wirt im Hochdurchsatz analysieren lassen, kommen beispielsweise in Frage 1,2-Dehydroreticulin-Reduktasen (1.5.1.27), 2-Enoyl-CoA-Reduktase (1.3.1.10), 2-Enoyl-CoA-Reduktasen (1.3.1.39), Alkenal/on-Oxidoreduktasen (1.3.1.74) Cytochrom-P450-Reduktase (1.6.2.4), NADP(H)-Dehydrogenasen (1.6.99.1), NADP(H)-Dehydrogenasen (Flavin) (1.6.8.2), NADP(H)-Dehydrogenasen (Quinon) (1.6.5.10), NADP(H)-abhängige 1,5-anhydro-D-Fruktose-Reduktasen (1.1.1.263), NADP(H)-abhängige Cytochrom-P450-Reduktasen (1.6.2.4), Diaphorasen (1.6.99.1), DT-Diaphorasen (1.6.5.5), Ferredoxin-Reduktasen (1.18.1.2), NADP(H)-Oxidasen (1.6.3.1, 1.6.5.10, 1.6.3.1, 1.6.3.1, 1.6.3.1), P450-Oxidoreduktase (1.6.2.4), P450-Reduktase (1.6.2.4), Peroxidase (1.11.1.2), Quinon-Acceptor-Oxidoreduktase (1.6.5.5), Quinon-Oxidoreduktase (1.6.5.10), NADP(H)-spezifische FMN-Reduktase (1.5.1.38), Thioredoxin-Reduktase (1.8.1.9), Transhydrogenase (1.6.1.2), NADP(H)-Aldehyd-Reduktase (1.1.1.2), Aldopentose-Reduktase (1.1.1.21), NADP(H)-Aldose-Reduktase (1.1.1.21), NADP(H)-Carbonyl-Reduktase (1.1.1.184), NADP(H)-CYP-Reduktase (1.6.2.4), NADP(H)-Cytochrom-c-Oxidoreduktase (1.6.2.4), NADP(H)-Cytochrome-c-Reduktase (1.1.1.2), NADP(H)-Cytochrome-f-Reduktase (1.6.2.5), NADP(H)-Cytochrom-P450-Reduktase (1.6.2.4) und die NADP(H)-Cytochrom-P450-Reduktase (1.14.13.68).

Die Plasmide, die Mutationen in Genen der NADP(H)-abhängigen Enzyme enthalten, werden anschließend durch Transformation in den Mikroorganismus, wie beispielsweise E. coli oder C. glutamicum, eingebracht. Der Begriff "Transformation" umfasst dabei sämtliche Methoden zur Übertragung von Polynukleotiden, insbesondere DNA, in ein gewünschtes Bakterium. Hierzu gehören unter anderem die Verwendung von isolierter DNA bei der Transformation, Elektrotransformation bzw. Elektroporation, die Übertragung durch Zellkontakt wie bei der Konjugation oder die Übertragung von DNA mittels Partikelbeschuss.

Nachdem im Verfahrensschritt (III) ein Gen, welches gegebenenfalls für ein NADP(H)-abhängiges Enzym kodiert, in einzelne Zellen einer Zellsuspension aus den im Verfahrensschritt (II) erhaltenen Zellen eingebracht (und exprimiert) worden ist, werden die Zellen dann im Verfahrensschritt (IV) mit einem Substrat für ein NADP(H)-anhängiges Enzym inkubiert und im Verfahrensschritt V) werden dann einzelne Zellen in der Zellsuspension mit erhöhter Aktivität NADP(H)-abhängiger Enzyme durch Detektion der intrazellulären Fluoreszenzaktivität identifiziert. Dazu wird die Zellsuspension elektromagnetischer Strahlung in derjenigen Frequenz ausgesetzt, welche das Autofluoreszenzprotein des NADP(H)-Nanosensors zur Emission von Licht anregt.

Im Verfahrensschritt VI) werden dann die identifizierten Zellen aus der Zellsuspension abgetrennt, wobei dieses Abtrennen vorzugsweise mittels Durchflusszytometrie (FACS = fluorescence activated cell sorting), ganz besonders bevorzugt mittels Hochdurchflusszytometrie (HT-FACS = high througput fluorescence activated cell sorting) erfolgt. Einzelheiten zur Analyse von Zellsuspensionen mittels Durchflusszytometrie können beispielsweise Sack U, Tarnok A, Rothe G (Hrsg): Zelluläre Diagnostik. Grundlagen, Methoden und klinische Anwendungen der Durchflusszytometrie, Basel, Karger, 2007, Seiten 27 - 70 entnommen werden.

Im Verfahrensschritt VII) werden dann die für ein NADP(H)-abhängiges Enzym kodierenden Gene in den identifizierten Zellen isoliert und gegebenenfalls analysiert, in dem beispielsweise die Enzyme-tragenden Plasmide aus den abgetrennten Zellen isoliert und deren Mutationen, die zu veränderter Fluoreszenz führen, durch Sequenzierung identifiziert und verifiziert werden.

Einen Beitrag zur Lösung der eingangs genannten Aufgaben leistet auch die Verwendung des NADP(H)-Nanosensors, wie vorstehend im Zusammenhang mit der erfindungsgemäßen Zelle beschrieben, zum Identifizieren von Genen, welche für ein NADP(H)-abhängiges Enzym kodieren, *in vivo.*

Die Erfindung wird nun anhand von Figuren und nicht limitierenden Beispielen näher erläutert.

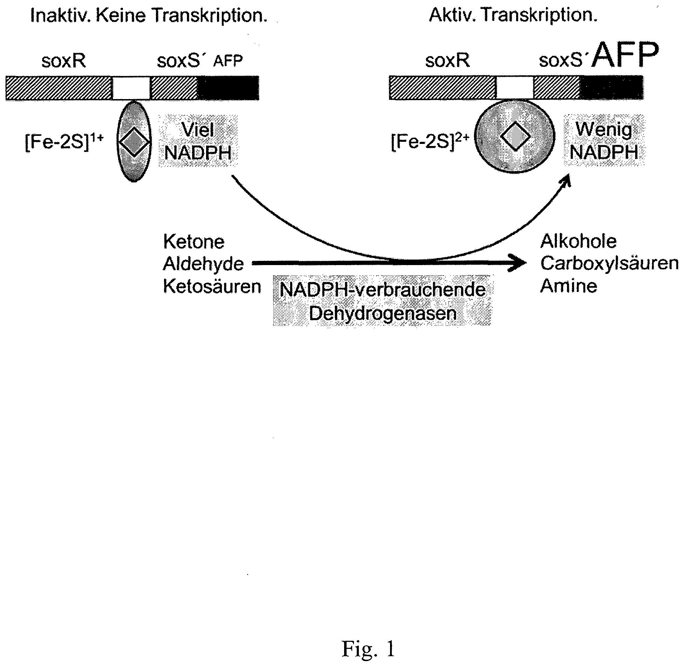

Es zeigt die Figur 1 die Funktionsweise des NADP(H)-Nanosensors am Beispiel der eingangs beschriebenen besonders besvorzugten Ausführungsform.

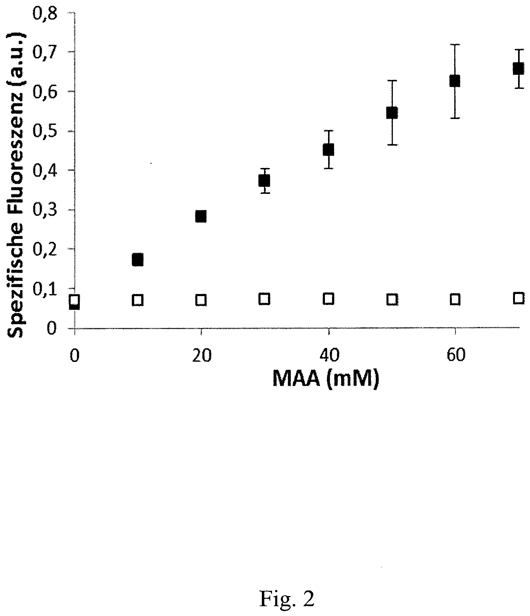

Es zeigt die Figur 2 die spezifische Fluoreszenz der im Beispiel 3 hergestellten *E. coli* BL21(DE3)-Zellen mit dem NADP(H)-Nanosensor (pSensox) und exprimierter Alkoholdehydrogenase (Lbadh) (geschlossene Quadrate). Die Fluoreszenz des Nanosensors pSennegK mit inaktiver Alkoholdehydrogenase ist als Kontrolle gezeigt (offene Quadrate).

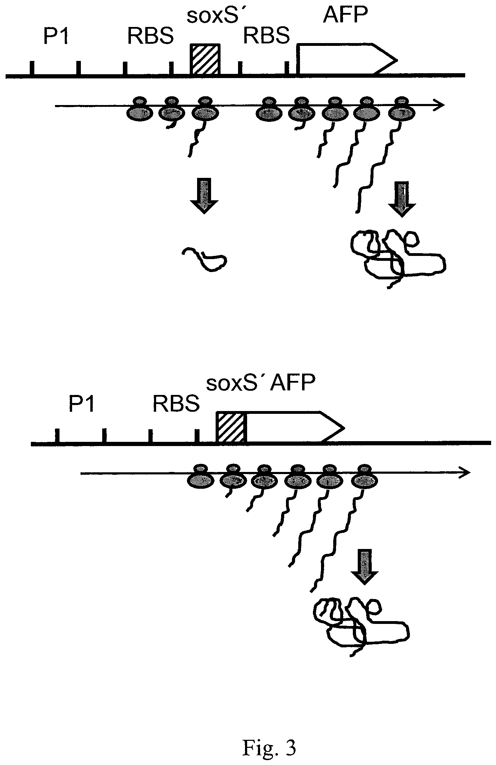

Es zeigt die Figur 3 schematisch die Bildung des Autofluoreszenzsproteins als transkriptionale (oben) und translationale (unten) Fusion.

Gemäß der Figur 1 kann der NADP(H)-Nanosensor das E. coli-Gen für SoxR (soxR), den sich daran anschließenden intergenischen Bereich aus E. coli, der zwischen soxR und soxS lokalisiert ist und der die SoxR-Bindesequenz und die sich an die SoxR-Bindesequenz anschließende soxS-Promotorsequenz umfasst, eine sich daran anschließende Teilsequenz des soxS-Gens aus E. coli (soxS') sowie ein sich daran anschließendes, unter der Kontrolle der soxS-Promotorsequenz befindliche Nukleinsäuresequenz, welche für ein Autofluoreszenzprotein kodiert (AFP), umfassen. Bei hoher cytosolischer NADP(H)-Konzentration (links oben in Figur 1) liegen die [2Fe-2S]-Cluster (Raute) von am Promoter gebundenen SoxR reduziert vor. Bei niedriger NADPH Verfügbarkeit (rechts oben in Figur 1) sind die [2Fe-2S]-Cluster oxidiert, und die resultierende Verwindung der soxS-Promoterregion ermöglicht der RNA-Polymerase die Transkriptionsinitiation des Zielgens. Das native Zielgen soxS ist erfindungsgemäß mit einem Autofluoreszenzprotein (AFP) fusioniert. NADP(H)-abhängige Enzyme bewirken durch Verbrauch von NADP(H) verstärkte Expression von soxS'-AFP und damit verstärkte Fluoreszenz von Zellen in Folge verstärkten NADP(H)-Verbrauchs.

Figur 3 zeigt oben die transkriptionale und unten die translationale Fusion. In beiden Fällen wird ein Transkript durch den Promoter P1 gebildet, der der durch SoxR kontrollierte soxS-Promoter ist. Während bei der transkriptionalen Fusion durch eine zweite Ribosomenbindestelle (RBS) zwei getrennte Peptide gebildet werden, wird bei der translationalen Fusion ein einziges Peptid gebildet, das Fusionsprotein, bei dem das Autofluoreszenzprotein zusätzliche Aminosäuresequenzen enthält.

### Beispiele

### Beispiel 1

### Konstruktion des NADPH-Nanosensors (transkriptionale Fusion)

Mit den Primerpaaren SoxS_for_SphI (SEQ.-ID.-Nr.04) und SoxR_rev_SalI (SEQ.-ID.-Nr. 05) sowie chromosomaler DNA von E. coli DH5α als Template wurde das Gen soxR zusammen mir der intergenischen Region von soxR-soxS und den ersten 63 Nukleotiden von soxS amplifiziert.
SoxS_for_SphI:
   ATCTGCATGCTTACGGCTGGTCAATATGCTCGTC
SoxR_rev_SalI:
   GCTAGTCGACCAAACTAAAGCGCCCTTGTG

Mit den Primerpaaren EYFP_for_SphI (SEQ.-ID.-Nr.06) und EYFP_rev_ClaI (SEQ.-ID.-Nr. 07) sowie dem Vektor pSenLys als Template wurde das Gen eyfp zusammen mit einer Ribosomenbindestelle amplifiziert. Der Vektor pSenLys ist in der Patentanmeldung WO-A-2011/138006 beschrieben.
EYFP_for_SphI:
   AGAGGCATGCAAGGAGAATTACATGGTGAGCAAGGGCGAGG
EYFP_rev_ClaI:
   GCGCATCGATTTATTACTTGTACAGCTCGTCCATG

Der Vektor pBtacLbadh kodiert für die NADPH-abhängige Alkoholdehydrogenase aus Lactobacillus brevis (Lbadh). Er ist bei Ernst et al. beschrieben (Ernst M, Kaup B, Müller M, Bringer-Meyer S, Sahm H, Appl. Microbiol. Biotechnol. 2005, 66(6), Seiten 629-34). Der Vektor pBtacLbadh wurde mit den Restriktionsenzymen SalI und ClaI behandelt, und das ∼ 5,0 kb große Vektorfragment aus dem Agarosegel isoliert und mit alkalischer Phosphatase behandelt und mit dem QIAquick Gel Extraction Kit (Cat.-Nr. 28704) der Firma Quiagen (Hilden, Deutschland) aufgereinigt. Anschließend wurden die beiden PCR-Produkte und der Vektor mit mittels T4-DNA-Ligase von New England BioLabs ligiert (New England Biolabs, 240 County Road, Ipswich, MA 01938-2723). Der Ligationsansatz wurde in den E. coli-Stamm DH5α transformiert. Die Selektion von Plasmidtragenden Zellen erfolgte durch Ausplattieren des Transformationsansatzes auf LB Agar (Sambrook et al.: "Molecular cloning: a laboratory manual", 2. Auflage, Cold Spring Harbor Laboratory Press, Cold Spring Harbor, N.Y.), der mit 50 mg/l Ampicillin supplementiert worden war. Plasmid-DNA wurde aus einer Transformante isoliert, durch Behandlung mit dem Restriktionsenzym BamHI mit anschließender Agarosegel-Elektrophorese überprüft. Das Plasmid wurde pSenSox genannt, und ist als Sequenz SEQ.-ID.-Nr. 08 hinterlegt.

Als Derivat mit veränderter Alkoholdehydrogenase, Lbadh, wurde pSennegK geschaffen. Dazu wurde mit den Primern ADH_negK_for (SEQ.-ID.-Nr. 09) und ADH_negK_rev (SEQ.-ID.-Nr. 10) und wiederum pBtacLbadh als Template eine inaktive Lbadh mit einer um 221 bp deletierter Alkoholdehydrogenase amplifiziert. Das resultierende Fragment wurde wie mit dem ∼ 5,7 kb großen Vektorfragment das Gen eyfp zusammen mit einer Ribosomenbindestelle enthielt, ligiert. Die Sequenz des resultierenden Vektors ist als SEQ.-ID.-Nr. 11 hinterlegt.
ADH_negK_for:
   ACAAGAATTCGCTAAGAGTGTCGGCACTCC
ADH_negK_rev:
   GGCCAAGCTTCCGAAGAAGACACCATCAAG

Als weiteres Derivat mit veränderter Alkoholdehydrogenase, Lbadh, wurde pSen-L194S geschaffen. Dazu wurde mit den Primern L194S_for (SEQ.-ID.-Nr. 12) und L194S_rev (SEQ.-ID.-Nr. 13) pSenSox als Template zur gerichteten Einführung der Mutation amplifiziert. Das erzeugte Plasmid wurde mittels Sequenzierung verifiziert. Die Sequenz des resultierenden Vektors ist als SEQ.-ID.-Nr. 14 hinterlegt.
L194S_for:
   CTGGCTACATCAAGACACCATCTGTTGATG
L194S_rev:
   CGGCCCCTGGTAGGTCATCAACAGATGGTG

Als weiteres Derivat mit veränderter Alkoholdehydrogenase, Lbadh, wurde pSen-L194A geschaffen. Dazu wurde mit den Primern L194A_for (SEQ.-ID.-Nr. 15) und L194A_rev (SEQ.-ID.-Nr. 16) pSenSox als Template zur gerichteten Einführung der Mutation amplifiziert. Das erzeugte Plasmid wurde mittels Sequenzierung verifiziert. Die Sequenz des resultierenden Vektors ist als SEQ.-ID.-Nr. 17 hinterlegt.
L194A_for:
   CTGGCTACATCAAGACACCAGCGGTTGATG
L194A_rev:
   CGGCCCCTGGTAGGTCATCAACCGCTGGTG

### Beispiel 2

### Nutzung des NADP(H)-Nanosensors zum Verfolgen Alkoholdehydrogenaseabhängiger Produktbildung

Mit dem Plasmid pSenSox wurde E. coli BL21(DE3) (Life Technologies GmbH, Frankfurter Straße 129B, 64293 Darmstadt) transformiert. Mit einer Einzelkolonie wurden 5 ml 2 × YT Medium (16 g/L Trypton, 10 g/L Hefeextrakt, 5 g/L NaCl) beimpft und die Kultur über Nacht bei 37°C und 130 UpM inkubiert. Mit dieser Vorkultur wurde die Hauptkultur zu einer OD von 0.05 in 50 mL 2 × TY angeimpft, die bei 37°C und 130 UpM inkubiert wurde. Bei der OD von 0.3 wurde 1 mM IPTG zugefügt und die Kultur für weitere 3 Stunden bis zu einer OD von 5-6 inkubiert.

Anschließend wurden je 0,9 mL der Zellsuspension in ein Reaktionsgefäß der Mikrotiterplatte Flowerplate (48-well) des BioLector Kultivierungssystems gegeben (m2plabs GmbH, Aachen, Germany). Zur Zellsuspension wurde Methylacetoacetat (MAA) in steigender Konzentration in einem konstanten Volumen von 0,1 mL zugefügt. Anschließend wurde die Mikrotiterplatte Flowerplate bei 30°C, 1200 UpM, Schüttelradius 3 mm inkubiert. In dem BioLector Kultivierungssystem wurde online das Wachstum als Streulicht bei 620 nm aufgezeichnet, sowie die Fluoreszenz der Kultur bei einer Anregungswellenlänge von 485 nm und einer Emissionswellenlänge von 520 nm kontinuierlich aufgezeichnet. Die spezifische Fluoreszenz nach 10 Stunden wurde gegenüber der Menge an zugegebenem MAA aufgetragen, und ist in Figur 2 dargestellt (es wurden 0 - 70 mM Methylacetoacetat zu einzelnen Ansätzen gegeben und nach 10 Stunden die spezifische Fluoreszenz bestimmt, die als schwarz gefüllte Quadrate angegeben sind; als Negativkontrolle (leere Quadrate) diente E. coli BL21(DE3) pSennegK mit inaktiver Lbadh.) Figur 2 zeigt eine Zunahme der Fluoreszenz bei zunehmender MAA-Konzentration. Diese Zunahme ist pSenSox bedingt, da eine Kontrollreaktion mit dem Plasmid pSennegK mit inaktiver Alkoholdehydrogenase, der aber sonst identisch ist zu pSenSox ist, keine Fluoreszenzzunahme bewirkt.

### Beispiel 3

### Nutzung des NADP(H)-Nanosensors zur Bestimmung unterschiedlicher Alkoholdehydrogenase Aktivitäten

Der Stamm E. coli BL21(DE3) (Life Technologies GmbH, Frankfurter Straße 129B, 64293 Darmstadt) wurde jeweils mit pSennegK, pSen-L194S, und pSen-L194A transformiert. Zusätzlich wurde der in Beispiel2 beschriebene Stamm E. coli BL21(DE3) pSenSox mit pET28a als zweitem Plasmid transformiert. Der letztgenannte Vektor wurde von Novagen (Life Technologies GmbH, Frankfurter Straße 129B, 64293 Darmstadt) bezogen. Mit einer Einzelkolonie des jeweiligen Stammes wurden 5 ml 2 × YT Medium (16 g/L Trypton, 10 g/L Hefeextrakt, 5 g/L NaCl) beimpft und die Kultur über Nacht bei 37°C und 130 UpM inkubiert. Mit dieser Vorkultur wurde die Hauptkultur zu einer OD von 0.05 in 50 mL 2xTY angeimpft, die bei 37°C und 130 UpM inkubiert wurde. Bei der OD von 0.3 wurde kein IPTG, bezw. dem Stamm E. coli BL21(DE3) pSenSox 1 mM IPTG zugefügt und die Kultur für weitere 3 Stunden bis zu einer OD von 5-6 inkubiert.

Anschließend wurden, wie in Beispiel 2 beschrieben, je 0,9 mL der Zellen in je ein Reaktionsgefäß der Mikrotiterplatte Flowerplate (48-well) des BioLector Kultivierungssystems gegeben (m2plabs GmbH, Aachen, Germany). Zur Zellsuspension wurde jeweils, in 0,1 mL, Methylacetoacetat (MAA) zu einer Endkonzentration von 40 mM zugefügt. Anschließend wurde die Mikrotiterplatte Flowerplate bei 30°C, 1200 UpM, Schüttelradius 3 mm inkubiert, und die spezifische Fluoreszenz bestimmt. Die nach 19 Stunden erhaltene spezifische Fluoreszenz ist in Tabelle 1 angegeben.

Zusätzlich wurde die Alkoholdehydrogenaseaktivität der rekombinanten E. coli-Zellen in den einzelnen Ansätzen bestimmt. Dazu wurden die Zellen bei 10.000 × g, 4°C, 5 min geerntet und in 100 mM Kaliumphosphatpuffer, pH 6,5, 1 mM Dithiothreitol, 1 mM MgCl₂ aufgenommen. Die Zellen wurden mittels des Silamat S5 (Ivoclar Vivadent GmbH, Germany) unter zuhilfenahme von Glaskugeln mit 0,1 mm Durchmesser aufgeschlossen. Der Rohextrakt der nach Zentrifugation bei 16.000 × g, 4°C, 20 min erhalten wurde, wurde im Enzymtest zur Quantifizierung der Alkoholdehydrogenase Aktivität eingesetzt. Der Test enthielt 5 mM Methylacetoacetat, 0,25 mM NADPH, and 1 mM MgCl₂ in 100 mM Kaliumphosphatpuffer, pH 6,5, und 0,01-0,1 mL Rohextrakt. Die Reduktion von NADP(H) wurde bei 340 nm und 30°C verfolgt. Eine Enzymeinheit (U) ist als diejenige Rohextraktmenge angegeben, die 0,001 mmol NADP(H) pro Minute reduziert. Sie ist ebenfalls in Tabelle 1 angeführt.

### Beispiel 4

### Isolation mutierter Alkoholdehydrogenase mit veränderter Substraterkennung

Die Alkoholdehydrogenase Lbadh aus Lactococcus lactis hat hohe Aktivität mit Methylacetoacetat, aber nur eine geringe Aktivität von etwa 10 % mit 4-Methyl-2-pentanon als Substrat. Um eine Lbadh mit höherer Aktivität zu evolvieren, wurden in pSenSox zufällige Mutationen durch error-prone PCR (epPCR) eingefügt. Zur Einführung der Mutationen wurden 10 ng pSenSox als Template pro Reaktion eingesetzt, sowie 0,1 - 0,8 mM Mn²⁺, wobei bei den niedrigeren Konzentrationen von unter < 0,2 mM Mn²⁺ mit Mg²⁺, eine Gesamtkonzentration von mindestens 0,2 mM eingestellt wurde. Pro Reaktion wurden 0,5 µl Taq-Polymerase von Fermentas (Katalog Nr. EP0401) zugesetzt. Als primer wurden die Polynukleotide
SEQ.-ID.-Nr. 18:
   ACAAGAATTCGCTAAGAGTGTCGGCACTCC
SEQ.-ID.-Nr. 19:
   GGCCAAGCTTCCGAAGAAGACACCATCAAG
benutzt. Die Reaktionen wurden 30 Minuten inkubiert. Anschließend wurden die Reaktionsprodukte mit BamHI und SalI behandelt, und mit dem zuvor ebenso behandelten Vektor pSenSox ligiert.

Mit den Ligationsprodukten wurde E. coli DH5amer transformiert (Grant, 1990, Proceedings of the National Academy of Sciences, USA, 87, Seiten 4645-4649). Nach Inkubation für 30 h wurden Transformanten mit 10 mL 2 × YT von den Platten abgeschwemmt, und zehnfach in frisches 2 × YT Medium verdünnt. Nach 4 Stunden Inkubation bei 37°C wurde 20 mM 4-Methyl-2-pentanon als Substrat zugesetzt und nach weiterer dreistündiger Inkubation der FACS Analyse und Sortierung zugeführt.

Zur FACS Analyse und Sortierung der Zellen mit hoher Fluoreszenz wurde die Zellsuspension in 2 × YT Medium auf eine optische Dichte unter 0.1 eingestellt, und unmittelbar dem FACS ARIA II high-speed cell sorter (Becton Dickinson GmbH, Tullastr. 8-12, 69126 Heidelberg) zugeführt. Die Analyse erfolgte mit den Anregungswellenlängen von 488 und 633 nm und die Detektion bei den Emissionswellenlängen von 530 ± 15 nm und 660 ±10 nm bei einem Probendruck von 70 psi. Die Daten wurden mit der zum Gerät gehörenden Software-Version BD DIVA 6.1.3 analysiert. Als Mantelflüssigkeit wurde BD FACSflow verwendet. Das elektronische gating wurde anhand des forward und backward scatters eingestellt, um nicht-bakterielle Partikel auszuschließen. Um EYFP-positive Zellen zu sortieren, wurde die nächste Stufe des elektronischen gatings gewählt, um nicht-fluoreszierende Zellen auszuschließen. Auf diese Weise wurden 123 fluoreszierende Zellen auf Petrischalen, die 2 × YT Medium enthielten, aussortiert.

Mit den nach Inkubation für 30 Stunden bei 37°C erhaltenen Kolonien wurden, wie in Beispiel 2 beschrieben, Reaktionsgefäße der Mikrotiterplatte Flowerplate (48-well) des BioLector Kultivierungssystems beimpft (m2plabs GmbH, Aachen, Germany). Als Substrat wurde aber nicht Methylacetoacetat, sondern 20 mM 4-Methyl-2-pentanon benutzt. Nach 120 Minuten wurde die spezifische Fluoreszenz quantifiziert, und ein Klon ausgewählt, dessen Alkoholdehydrogenaseaktivität im Enzymtest, wie in Beispiel 3 beschrieben, bestimmt wurde. Als Substrat wurde dabei 20 mM 4-Methyl-2-pentanon benutzt.

Die so erhaltene Mutante mit dem Plasmid pSen-A93M hat eine um 26 % gesteigerte spezifische Aktivität gegenüber dem Ausgangsstamm (Tabelle 2), sowie eine um 37 % erhöhte Umsatzgeschwindigkeit mit 4-Methyl-2-pentanon als Substrat. Die Sequenz des Plasmids pSen-A93M ist als SEQ.-ID.-Nr. 20 hinterlegt.

**Tabelle 1: Korrelation der Alkoholdehydrogenaseaktivität ganzer Zellen mit der spezifischen Fluoreszenz.**

| Stamm | IPTG | Alkoholdehydrogenase Aktivität (U mg⁻¹) | Spezifische Fluoreszenz |
|---|---|---|---|
| BL21(DE3) pSennegK | - | 0,03 ± 0,01 | 0,06 |
| BL21(DE3) pSenSox, pET28a | - | 0,5 ± 0,1 | 0,09 |
| BL21(DE3) pSenL194S | - | 0,7 ± 0,3 | 0,11 |
| BL21(DE3) pSenL194A | - | 2,7 ± 0,6 | 0,17 |
| BL21(DE3) pSenSox | - | 6,2 ± 0,6 | 0,38 |
| BL21(DE3) pSenSox | + | 15,2 ± 2,0 | 0,45 |

**Tabelle 2: Steigerung der Aktivität und Umsatzrate der mittels NADP(H)-Nanosensor und FACS isolierten Alkoholdehydrogenase mit 4-Methyl-2-pentanon als Substrat.**

| Stamm | AlkoholdehydrogenaseAktivität (U mg-1) | vₘₐₓ (U mg-1) | K_{M} (mM) |
|---|---|---|---|
| DH5α pSensox | 1,9 ± 0,2 | 1,9 ± 0,02 | 0,10 ± 0,01 |
| DH5α pSenA93M | 2,4 ± 0,1 | 2,6 ± 0,03 | 0,88 ± 0,03 |

### Beispiel 5

### Konstruktion des NADPH-Nanosensors (translationale Fusion)

Mit den Primerpaaren SoxS_for_SphI t1 (SEQ.-ID.-Nr. 21) und SoxR_rev_SalI_t1 (SEQ.-ID.-Nr. 22) sowie chromosomaler DNA von E. coli DH5α als Template wurde das Gen soxR zusammen mit der intergenischen Region von soxR-soxS und den ersten 63 Nukleotiden von soxS amplifiziert.
SoxS_for_SphI_t1:
   ATCTGCATGCCGGCTGGTCAATATGCTCGTC
SoxR_rev_SalI_t1:
   GCTAGTCGACCAAACTAAAGCGCCCTTGTG

Mit den Primerpaaren EYFP_for_SphI_t1 (SEQ.-ID.-Nr.23) und EYFP_rev_ClaI_t1 (SEQ.-ID.-Nr. 24) sowie dem Vektor pSenLys als Template wurde das Gen eyfp amplifiziert. Der Vektor pSenLys ist in der Patentanmeldung WO-A-2011/138006 beschrieben.
EYFP _for_SphI_t1:
   AGAGGCATGCGTGAGCAAGGGCGAGG
EYFP_rev_ClaI_t1:
   GCGCATCGATTTATTACTTGTACAGCTCGTCCATG

Der Vektor pBtacLbadh kodiert für die NADPH-abhängige Alkoholdehydrogenase aus Lactobacillus brevis (Lbadh). Er ist bei Ernst et al. beschrieben (Ernst M, Kaup B, Müller M, Bringer-Meyer S, Sahm H, Appl. Microbiol. Biotechnol. 2005, 66(6), Seiten 629-34). Der Vektor pBtacLbadh wurde mit den Restriktionsenzymen SalI und ClaI behandelt, und das ∼ 5,0 kb große Vektorfragment aus dem Agarosegel isoliert und mit alkalischer Phosphatase behandelt und mit dem QIAquick Gel Extraction Kit (Cat.-Nr.28704) der Firma Quiagen (Hilden, Deutschland) aufgereinigt. Anschließend wurden die beiden PCR-Produkte und der Vektor mit mittels T4-DNA-Ligase von New England BioLabs ligiert (New England Biolabs, 240 County Road, Ipswich, MA 01938-2723). Der Ligationsansatz wurde in den E. coli-Stamm DH5α transformiert. Die Selektion von Plasmidtragenden Zellen erfolgte durch Ausplattieren des Transformationsansatzes auf LB Agar (Sambrook et al.: "Molecular cloning: a laboratory manual", 2. Auflage, Cold Spring Harbor Laboratory Press, Cold Spring Harbor, N.Y.), der mit 50 mg/l Ampicillin supplementiert worden war. Plasmid-DNA wurde aus einer Transformante isoliert, durch Behandlung mit dem Restriktionsenzym BamHI mit anschließender Agarosegel-Elektrophorese überprüft. Das Plasmid wurde pSenSox_t1 genannt, und ist als Sequenz SEQ.-ID.-Nr. 25 hinterlegt.

### SEQUENZEN

SEQ.-ID.-NR. 01
SEQ.-ID.-NR.02
SEQ.-ID.-NR.03
SEQ.-ID.-NR.04
   atctgcatgc ttacggctgg tcaatatgct cgtc 34
SEQ.-ID.-NR. 05
   gctagtcgac caaactaaag cgcccttgtg 30
SEQ.-ID.-NR. 06
   agaggcatgc aaggagaatt acatggtgag caagggcgag g 41
SEQ.-ID.-NR. 07
   gcgcatcgat ttattacttg tacagctcgt ccatg 35
SEQ.-ID.-NR. 08
SEQ.-ID.-NR. 09
   acaagaattc gctaagagtg tcggcactcc 30
SEQ.-ID.-NR. 10
   ggccaagctt ccgaagaaga caccatcaag 30
SEQ.-ID.-NR. 11
SEQ.-ID.-NR. 12
   ctggctacat caagacacca tctgttgatg 30
SEQ.-ID.-NR. 13
   cggcccctgg taggtcatca acagatggtg 30
SEQ.-ID.-NR. 14
SEQ.-ID.-NR. 15
   ctggctacat caagacacca gcggttgatg 30
SEQ.-ID.-NR. 16
   cggcccctgg taggtcatca accgctggtg 30
SEQ.-ID.-NR. 17
SEQ.-ID.-NR. 18
   acaagaattc gctaagagtg tcggcactcc 30
SEQ.-ID.-NR. 19
   ggccaagctt ccgaagaaga caccatcaag 30
SEQ.-ID.-NR. 20
SEQ.-ID.-NR.21
   atctgcatgc cggctggtca atatgctcgt c 31
SEQ.-ID.-NR. 22
   gctagtcgac caaactaaag cgcccttgtg 30
SEQ.-ID.-NR. 23
   agaggcatgc gtgagcaagg gcgagg 26
SEQ.-ID.-NR. 24
   gcgcatcgat ttattacttg tacagctcgt ccatg 35
SEQ.-ID.-NR. 25

## Patentansprüche

1. Eine Zelle, umfassend einen NADP(H)-Nanosensor, wobei der NADP(H)-Nanosensor
i) eine Nukleinsäuresequenz, an die ein Regulator zu binden vermag, wobei der Oxidationszustand des Regulators von der NADP(H)-Verfügbarkeit abhängig ist;
ii) eine sich an die Nukleinsäuresequenz i) anschließende Promotorsequenz, an die eine RNA-Polymerase zu binden vermag, wobei die Affinität der RNA-Polymerase für die Promotorsequenz durch den Oxidationszustand des Regulators beeinflusst wird;
iii) eine sich unter der Kontrolle der Promotorsequenz ii) befindliche Nukleinsäuresequenz, die für ein Autofluoreszenzprotein kodiert,
umfasst, wobei die Nukleinsäuresequenz i) die SoxR-Bindesequenz, der Regulator der Sox-Regulator (SoxR) und die Promotorsequenz die soxS-Promotorsequenz ist und
wobei die Zelle weiterhin ein Plasmid mit einem gegebenenfalls mutierten Gen, welches für ein NADP(H)-abhängiges Enzym kodiert, umfasst.

2. Zelle nach Anspruch 1, wobei die Komponenten i) und ii) durch den intergenischen Bereich aus E. coli, der zwischen soxR und soxS lokalisiert ist und der die SoxR-Bindesequenz, die sich an die SoxR-Bindesequenz anschließende soxS-Promotorsequenz und eine sich an die soxS-Promotorsequenz anschließende Sequenz, die auf der Ebene der mRNA einer Ribosomenbindestelle entspricht, umfasst, oder durch eine hierzu homologe Nukleinsäuresequenz gebildet werden.

3. Zelle nach Anspruch 2, wobei die Komponenten i) und ii) gebildet werden durch eine Nukleinsäuresequenz ausgewählt aus der Gruppe bestehend aus:
a) einer Nukleinsäuresequenz gemäß SEQ.-ID.-Nr. 01,
b) einer Nukleinsäuresequenz, die eine Identität von mindestens 70 % zur Nukleinsäuresequenz von a) aufweist, wobei die Nukleinsäuresequenz in der Lage ist, SoxR dergestalt zu binden, dass die Affinität der RNA-Polymerase für den soxS-Promotor vom Oxidationszustand von SoxR abhängig ist, und
c) einer Nukleinsäuresequenz, die unter stringenten Bedingungen mit einer komplementären Nukleinsäuresequenz nach a) oder b) zu hybridisieren vermag, wobei die Nukleinsäuresequenz in der Lage ist, SoxR dergestalt zu binden, dass die Affinität der RNA-Polymerase für den soxS-Promotor vom Oxidationszustand von SoxR abhängig ist.

4. Zelle nach einem der vorhergehenden Ansprüche, wobei der NADP(H)-Nanosensor
(α1) das E. coli-Gen für SoxR (soxR) oder eine hierzu homologe Nukleinsäuresequenz;
(α2) den sich an (α1) anschließenden intergenischen Bereich aus E. coli, der zwischen soxR und soxS lokalisiert ist und der die SoxR-Bindesequenz, die sich an die SoxR-Bindesequenz anschließende soxS-Promotorsequenz und eine sich an die soxS-Promotorsequenz anschließende Sequenz, die auf der Ebene der mRNA einer Ribosomenbindestelle entspricht, umfasst, oder eine hierzu homologe Nukleinsäuresequenz, als Komponenten i) und ii);
(α3) gegebenenfalls eine sich an (α2) anschließende Teilsequenz des soxS-Gens aus E. coli oder eine hierzu homologe Nukleinsäuresequenz;
(α4) eine sich an (α2) oder (α3) anschließende und unter der Kontrolle der soxS-Promotorsequenz befindliche Nukleinsäuresequenz, welche für ein Autofluoreszenzprotein kodiert, als Komponente iii) umfasst.

5. Zelle nach einem der Ansprüche 1 bis 3, wobei der NADP(H)-Nanosensor
(β1) das E. coli-Gen für SoxR (soxR) oder eine hierzu homologe Nukleinsäuresequenz;
(β2) den sich an (β1) anschließenden intergenischen Bereich aus E. coli, der zwischen soxR und soxS lokalisiert ist und der die SoxR-Bindesequenz, die sich an die SoxR-Bindesequenz anschließende soxS-Promotorsequenz und eine sich an die soxS-Promotorsequenz anschließende Sequenz, die auf der Ebene der mRNA einer Ribosomenbindestelle entspricht, umfasst, oder eine hierzu homologe Nukleinsäuresequenz, wie vorstehend definiert, als Komponenten i) und ii);
(β3) die sich an (β2) anschließende und unter der Kontrolle der soxS-Promotorsequenz befindliche Sequenz des soxS-Gens aus E. coli, eine Teilsequenz dieses Gens oder einer hierzu homologe Nukleinsäuresequenz;
(β3') eine weitere, sich an (β3) anschließende Sequenz, die auf mRNA-Ebene einer Ribosomenbindestelle entspricht;
(β4) eine sich an (β3') anschließende und unter der Kontrolle der soxS-Promotorsequenz befindliche Nukleinsäuresequenz, welche für ein Autofluoreszenzprotein kodiert, als Komponente iii) umfasst.

6. Zelle nach Anspruch 4 oder 5, wobei die Komponente (α1) bzw. (β1) ausgewählt ist aus der Gruppe bestehend aus:
a) einer Nukleinsäuresequenz gemäß SEQ.-ID.-Nr. 02,
b) einer Nukleinsäuresequenz, kodierend ein Polypeptid mit einer Aminosäuresequenz gemäß SEQ.-ID.-Nr. 03,
c) einer Nukleinsäuresequenz, die eine Identität von mindestens 70 % zur Nukleinsäuresequenz von a) oder b) aufweist, wobei die Nukleinsäuresequenz ein Polypeptid kodiert, welches an die SoxR-Bindesequenz im intergenischen Bereich aus E. coli, der zwischen soxR und soxS lokalisiert ist, zu binden vermag und dessen Oxidationszustand die Affinität der RNA-Polymerase für die ebenfalls im intergenischen Bereich aus E. coli lokalisierte Promotorsequenz zu beeinflussen vermag,
d) einer Nukleinsäuresequenz, kodierend ein Polypeptid, das eine Homologie von mindestens 70 % zur SEQ.-ID.-Nr. 03 aufweist, wobei die Nukleinsäuresequenz ein Polypeptid kodiert, welches an die SoxR-Bindesequenz im intergenischen Bereich aus E. coli, der zwischen soxR und soxS lokalisiert ist, zu binden vermag und dessen Oxidationszustand die Affinität der RNA-Polymerase für die ebenfalls im intergenischen Bereich aus E. coli lokalisierte Promotorsequenz zu beeinflussen vermag, und
e) einer Nukleinsäuresequenz, die unter stringenten Bedingungen mit einer komplementären Nukleinsäuresequenz nach einer der Gruppen a) bis d) zu hybridisieren vermag, wobei die Nukleinsäuresequenz ein Polypeptid kodiert, welches an die SoxR-Bindesequenz im intergenischen Bereich aus E. coli, der zwischen soxR und soxS lokalisiert ist, zu binden vermag und dessen Oxidationszustand die Affinität der RNA-Polymerase für die ebenfalls im intergenischen Bereich aus E. coli lokalisierte Promotorsequenz zu beeinflussen vermag.

7. Zelle nach einem der vorhergehenden Ansprüche, wobei die Nukleinsäuresequenz (iii), die für ein Autofluoreszenzprotein kodiert, ausgewählt ist aus der Gruppe bestehend aus den Genen kodierend für das Green Fluorescent Protein (GFP), das Yellow Fluorescent Protein (YFP) das Blue Fluorescent Protein (BFP), das Cyan Fluorescent Protein (CFP), das enhanced Green Fluorescent Protein (EGFP), das enhanced Yellow Fluorescent Protein (EYFP), das enhanced Blue Fluorescent Protein (EBFP), das enhanced Cyan Fluorescent Protein (ECFP), DsRed, HcRed, AsRed, AmCyan, ZsGreen, AcGFP und ZsYellow. Ebenso kann ein Photoreceptorprotein benutzt werden, das eine sogenannte LOV Domäne enthält.

8. Zelle nach einem der vorhergehenden Ansprüche, wobei die Zelle ausgewählt ist aus der Gruppe bestehend aus Escherichia coli, Pseudomonas fluorescens, Corynebacterium glutamicum, Bacillus subtilis oder Saccharomyces cerevisiae.

9. Zelle nach einem der vorhergehenden Ansprüche, wobei das NADP(H)-abhängige Enzym ausgewählt ist aus der Gruppe bestehend aus Alkoholdehydrogenasen, Aldehyddehydrogenasen, Laktatdehoydrogenasen, Enoatreduktasen Epoxidreduktasen, Diaminopimelatdehydrogenasen, Aminosäuredehydrogenasen, Aldehydoxidoreduktasen, Alkanreduktasen, Aminreduktasen, Epoxiddehydrogenasen, Carboxylsäuredehydrogenasen, Hydroxysäureketoreduktasen und Hydroxysäuredehalogenasen.

10. Ein Verfahren zum Isolieren von Genen, welche für NADP(H)-abhängige Enzyme kodieren, umfassend die Verfahrensschritte:
(I) Bereitstellung eines NADP(H)-Nanosensors, wie in einem der Ansprüche 1 bis 7 definiert;
(II) Einbringen des NADP(H)-Nanosensors in eine Zelle;
(III) Einbringen eines Gens, welches gegebenenfalls für ein NADP(H)-abhängiges Enzym kodiert, in einzelne Zellen einer Zellsuspension aus den im Verfahrensschritt (II) erhaltenen Zellen;
(IV) Inkubieren der Zellen mit einem Substrat für das NADP(H)-anhängige Enzym;
(V) Identifizieren einzelner Zellen in der Zellsuspension mit erhöhter Aktivität NADP(H)-abhängiger Enzyme durch Detektion der intrazellulären Fluoreszenzaktivität;
(VI) Abtrennen der identifizierten Zellen aus der Zellsuspension;
(VII) Isolierung der für ein NADP(H)-abhängiges Enzym kodierenden Gene in den identifizierten Zellen.

11. Verwendung eines NADP(H)-Nanosensors, wie in einem der Ansprüche 1 bis 7 definiert, zum Identifizieren von Genen, welche für ein NADP(H)-abhängiges Enzym kodieren, in vivo.

## Claims

1. A cell comprising an NADP(H) nanosensor, said NADP(H) nanosensor comprising
i) a nucleic acid sequence capable of binding to a regulator, wherein the oxidation state of said regulator is a function of NADP(H) availability;
ii) a promoter sequence downstream from the nucleic acid sequence i), to which promoter sequence an RNA polymerase is able to bind, wherein the affinity of said RNA polymerase for the promoter sequence is influenced by the oxidation state of the regulator;
iii) a nucleic acid sequence coding for an auto-fluorescent protein, which nucleic acid sequence is under the control of the promoter sequence ii),
wherein the nucleic acid sequence i) is the SoxR-binding sequence, the regulator is the Sox regulator (SoxR), and the promoter sequence is the soxS promoter sequence, and
wherein the cell furthermore comprises a plasmid harbouring an optionally mutated gene coding for an NADP(H)-dependent enzyme.

2. Cell according to Claim 1, wherein components i) and ii) are formed by the *E. coli* intergenic region located between soxR and soxS and comprising the SoxR-binding sequence, the soxS promoter sequence downstream from the SoxR-binding sequence, and a sequence downstream from the soxS promoter sequence, which at the mRNA level corresponds to a ribosome-binding site, or by a nucleic acid sequence homologous thereto.

3. Cell according to Claim 2, wherein components i) and ii) are formed by a nucleic acid sequence selected from the group consisting of:
a) a nucleic acid sequence set forth in SEQ ID No. 01,
b) a nucleic acid sequence which is at least 70 % identical to the nucleic acid sequence of a), wherein the nucleic acid sequence is capable of binding SoxR such that the affinity of RNA polymerase for the soxS promoter is a function of the oxidation state of SoxR, and
c) a nucleic acid sequence which is able to hybridize under stringent conditions with a complementary nucleic acid sequence according to a) or b), wherein the nucleic acid sequence is capable of binding SoxR such that the affinity of RNA polymerase for the soxS promoter is a function of the oxidation state of SoxR.

4. Cell according to any of the preceding claims, wherein the NADP(H) nanosensor comprises
(α1) the *E. coli* SoxR gene (soxR) or a nucleic acid sequence homologous thereto;
(α2) downstream from (α1), the *E. coli* intergenic region located between soxR and soxS and comprising the SoxR-binding sequence, the soxS promoter sequence downstream from the SoxR-binding sequence, and a sequence downstream from the soxS promoter sequence, which at the mRNA level corresponds to a ribosome-binding site, or a nucleic acid sequence homologous thereto, as components i) and ii);
(α3) optionally a partial sequence of the *E. coli* soxS gene downstream from (α2) or a nucleic acid sequence homologous thereto;
(α4) a nucleic acid sequence downstream from (α2) or (α3) and under the control of the soxS promoter sequence, which codes for an auto-fluorescent protein, as component iii).

5. Cell according to any of Claims 1 to 3, wherein the NADP(H) nanosensor comprises
(β1) the *E. coli* SoxR gene (soxR) or a nucleic acid sequence homologous thereto;
(β2) downstream from (β1), the *E. coli* intergenic region located between soxR and soxS and comprising the SoxR-binding sequence, the soxS promoter sequence downstream from the SoxR-binding sequence, and a sequence downstream from the soxS promoter sequence, which at the mRNA level corresponds to a ribosome-binding site, or a nucleic acid sequence homologous thereto, as defined above, as components i) and ii);
(β3) the *E. coli* soxS gene sequence downstream from (β2) and under the control of the soxS promoter sequence, a partial sequence of this gene or of a nucleic acid sequence homologous thereto;
(β3') another sequence downstream from (β3), which at the mRNA level corresponds to a ribosome-binding site;
(β4) a nucleic acid sequence downstream from (β3') and under the control of the soxS promoter sequence, which codes for an auto-fluorescent protein, as component iii).

6. Cell according to Claim 4 or 5, wherein components (α1) and (β1), respectively, are selected from the group consisting of:
a) a nucleic acid sequence set forth in SEQ ID No. 02,
b) a nucleic acid sequence encoding a polypeptide having an amino acid sequence set forth in SEQ ID No. 03,
c) a nucleic acid sequence which is at least 70 % identical to the nucleic acid sequence of a) or b), wherein the nucleic acid sequence encodes a polypeptide which is able to bind to the SoxR-binding sequence in the *E. coli* intergenic region located between soxR and soxS and the oxidation state of which can influence the affinity of RNA polymerase for the promoter sequence likewise located in the *E. coli* intergenic region,
d) a nucleic acid sequence encoding a polypeptide which is at least 70 % homologous to SEQ ID No. 03, wherein the nucleic acid sequence encodes a polypeptide which is able to bind to the SoxR-binding sequence in the *E. coli* intergenic region located between soxR and soxS and the oxidation state of which can influence the affinity of RNA polymerase for the promoter sequence likewise located in the *E. coli* intergenic region, and
e) a nucleic acid sequence which is able to hybridize under stringent conditions with a complementary nucleic acid sequence according to any of groups a) to d), wherein the nucleic acid sequence encodes a polypeptide which is able to bind to the SoxR-binding sequence in the *E. coli* intergenic region located between soxR and soxS and the oxidation state of which can influence the affinity of RNA polymerase for the promoter sequence likewise located in the *E. coli* intergenic region.

7. Cell according to any of the preceding claims, wherein the nucleic acid sequence (iii) coding for an auto-fluorescent protein is selected from the group consisting of the genes coding for green fluorescent protein (GFP), yellow fluorescent protein (YFP), blue fluorescent protein (BFP), cyan fluorescent protein (CFP), enhanced green fluorescent protein (EGFP), enhanced yellow fluorescent protein (EYFP), enhanced blue fluorescent protein (EBFP), enhanced cyan fluorescent protein (ECFP), DsRed, HcRed, AsRed, AmCyan, ZsGreen, AcGFP and ZsYellow. A photoreceptor protein containing what is known as LOV domain may also be utilized.

8. Cell according to any of the preceding claims, wherein the Cell according to any of the preceding claims, wherein the cell is selected from the group consisting of Escherichia coli, *Pseudomonas fluorescens,* Corynebacterium glutamicum, *Bacillus subtilis* or *Saccharomyces cerevisiae.*

9. Cell according to any of the preceding claims, wherein the NADP(H)-dependent enzyme is selected from the group consisting of alcohol dehydrogenases, aldehyde dehydrogenases, lactate dehoydrogenases, enoate reductases, epoxide reductases, diaminopimelate dehydrogenases, amino acid dehydrogenases, aldehyde oxidoreductases, alkane reductases, amine reductases, epoxide dehydrogenases, carboxylic acid dehydrogenases, hydroxy acid ketoreductases and hydroxy acid dehalogenases.

10. A method for isolating genes coding for NADP(H)-dependent enzymes, said method comprising the steps of:
(I) providing an NADP(H) nanosensor as defined in any of Claims 1 to 7;
(II) introducing the NADP(H) nanosensor into a cell;
(III) introducing a gene which optionally codes for an NADP(H)-dependent enzyme into individual cells of a cell suspension of the cells obtained in step (II);
(IV) incubating the cells with a substrate for the NADP(H)-dependent enzyme;
(V) identifying individual cells in the cell suspension with increased activity of NADP(H)-dependent enzymes by detecting intracellular fluorescence activity;
(VI) removing the identified cells from the cell suspension;
(VII) isolating the genes coding for an NADP(H)-dependent enzyme in the identified cells.

11. Use of an NADP(H) nanosensor as defined in any of Claims 1 to 7 for identifying *in vivo* genes coding for an NADP(H)-dependent enzyme.

## Revendications

1. Cellule, comprenant un nanocapteur de NADP(H), le nanocapteur de NADP(H) comprenant :
i) une séquence d'acides nucléiques, à laquelle un régulateur peut se lier, l'état d'oxydation du régulateur étant dépendant de la disponibilité de NADP(H) ;
ii) une séquence de promoteur raccordée à la séquence d'acides nucléiques i), à laquelle une ARN-polymérase peut se lier, l'affinité de l'ARN-polymérase pour la séquence de promoteur étant influencée par l'état d'oxydation du régulateur ;
iii) une séquence d'acides nucléiques se trouvant sous le contrôle de la séquence de promoteur ii), qui code pour une protéine auto-fluorescente,
la séquence d'acides nucléiques i) étant la séquence de liaison à SoxR, le régulateur étant le régulateur Sox (SoxR) et la séquence de promoteur étant la séquence de promoteur soxS, et
la cellule comprenant en outre un plasmide contenant un gène éventuellement muté, qui code pour une enzyme dépendante de NADP(H).

2. Cellule selon la revendication 1, dans laquelle les composants i) et ii) sont formés par la zone intergénique d'E. coli, qui est localisée entre soxR et soxS, et qui comprend la séquence de liaison à SoxR, la séquence de promoteur soxS raccordée à la séquence de liaison à SoxR et une séquence raccordée à la séquence de promoteur soxS, qui correspond sur le plan de l'ARNm à un emplacement de liaison ribosomique, ou par une séquence d'acides nucléiques homologue.

3. Cellule selon la revendication 2, dans laquelle les composants i) et ii) sont formés par une séquence d'acides nucléiques choisie dans le groupe constitué par :
a) une séquence d'acides nucléiques selon SEQ ID NO : 1,
b) une séquence d'acides nucléiques qui présente une identité d'au moins 70 % avec la séquence d'acides nucléiques de a), la séquence d'acides nucléiques étant apte à se lier à SoxR de sorte que l'affinité de l'ARN-polymérase pour le promoteur soxS soit dépendante de l'état d'oxydation de SoxR, et
c) une séquence d'acides nucléiques qui peut être hybridée dans des conditions stringentes avec une séquence d'acides nucléiques complémentaire selon a) ou b), la séquence d'acides nucléiques étant apte à se lier à SoxR de sorte que l'affinité de l'ARN-polymérase pour le promoteur soxS soit dépendante de l'état d'oxydation de SoxR.

4. Cellule selon l'une quelconque des revendications précédentes, dans laquelle le nanocapteur de NADP(H) comprend :
(α1) le gène d'E. coli pour SoxR (soxR) ou une séquence d'acides nucléiques homologue ;
(α2) la zone intergénique d'E. coli raccordée à (α1), qui est localisée entre soxR et soxS, et qui comprend la séquence de liaison à SoxR, la séquence de promoteur soxS raccordée à la séquence de liaison à SoxR et une séquence raccordée à la séquence de promoteur soxS, qui correspond sur le plan de l'ARNm à un emplacement de liaison ribosomique, ou une séquence d'acides nucléiques homologue, en tant que composants i) et ii) ;
(α3) éventuellement une séquence partielle du gène soxS d'E. coli raccordée à (α2) ou une séquence d'acides nucléiques homologue ;
(α4) une séquence d'acides nucléiques raccordée à (α2) et (α3) et se trouvant sous le contrôle de la séquence de promoteur soxS, qui code pour une protéine auto-fluorescente, en tant que composant iii).

5. Cellule selon l'une quelconque des revendications 1 à 3, dans laquelle le nanocapteur de NADP(H) comprend :
(β1) le gène d'E. coli pour SoxR (soxR) ou une séquence d'acides nucléiques homologue ;
(β2) la zone intergénique d'E. coli raccordée à (β1), qui est localisée entre soxR et soxS, et qui comprend la séquence de liaison à SoxR, la séquence de promoteur soxS raccordée à la séquence de liaison à SoxR et une séquence raccordée à la séquence de promoteur soxS, qui correspond sur le plan de l'ARNm à un emplacement de liaison ribosomique, ou une séquence d'acides nucléiques homologue, telle que définie précédemment, en tant que composants i) et ii) ;
(β3) la séquence du gène soxS d'E. coli raccordée à (β2) et se trouvant sous le contrôle de la séquence de promoteur soxS, une séquence partielle de ce gène ou une séquence d'acides nucléiques homologue ;
(β3') une séquence supplémentaire raccordée à (β3), qui correspond sur le plan de l'ARNm à un emplacement de liaison ribosomique ;
(β4) une séquence d'acides nucléiques raccordée à (β3') et se trouvant sous le contrôle de la séquence de promoteur soxS, qui code pour une protéine auto-fluorescente, en tant que composant iii).

6. Cellule selon la revendication 4 ou 5, dans laquelle le composant (α1) ou (β1) est choisi dans le groupe constitué par :
a) une séquence d'acides nucléiques selon SEQ ID NO : 2,
b) une séquence d'acides nucléiques codant pour un polypeptide ayant une séquence d'acides aminés selon SEQ ID NO : 3,
c) une séquence d'acides nucléiques qui présente une identité d'au moins 70 % avec la séquence d'acides nucléiques de a) ou b), la séquence d'acides nucléiques codant pour un polypeptide qui peut se lier à la séquence de liaison à SoxR dans la zone intergénique d'E. coli, qui est localisée entre soxR et soxS, et dont l'état d'oxydation peut influencer l'affinité de l'ARN-polymérase pour la séquence de promoteur également localisée dans la zone intergénique d'E. coli,
d) une séquence d'acides nucléiques codant pour un polypeptide qui présente une homologie d'au moins 70 % avec la SEQ ID NO : 3, la séquence d'acides nucléiques codant pour un polypeptide qui peut se lier à la séquence de liaison à SoxR dans la zone intergénique d'E. coli, qui est localisée entre soxR et soxS, et dont l'état d'oxydation peut influencer l'affinité de l'ARN-polymérase pour la séquence de promoteur également localisée dans la zone intergénique d'E. coli, et
e) une séquence d'acides nucléiques qui peut être hybridée dans des conditions stringentes avec une séquence d'acides nucléiques complémentaire selon l'un des groupes a) à d), la séquence d'acides nucléiques codant pour un polypeptide qui peut se lier à la séquence de liaison à SoxR dans la zone intergénique d'E. coli, qui est localisée entre soxR et soxS, et dont l'état d'oxydation peut influencer l'affinité de l'ARN-polymérase pour la séquence de promoteur également localisée dans la zone intergénique d'E. coli.

7. Cellule selon l'une quelconque des revendications précédentes, dans laquelle la séquence d'acides nucléiques (iii), qui code pour une protéine auto-fluorescente, est choisie dans le groupe constitué par les gènes codant pour la Green Fluorescent Protein (GFP), la Yellow Fluorescent Protein (YFP), la Blue Fluorescent Protein (BFP), la Cyan Fluorescent Protein (CFP), la Enhanced Green Fluorescent Protein (EGFP), la Enhanced Yellow Fluorescent Protein (EYFP), la Enhanced Blue Fluorescent Protein (EBFP), la Enhanced Cyan Fluorescent Protein (ECFP), DsRed, HcRed, AsRed, AmCyan, ZsGreen, AcGFP et ZsYellow ; une protéine de photorécepteur qui contient un domaine dit LOV pouvant également être utilisée.

8. Cellule selon l'une quelconque des revendications précédentes, dans laquelle la cellule est choisie dans le groupe constitué par Escherichia coli, Pseudomonas fluorescens, Corynebacterium glutamicum, Bacillus subtilis ou Saccharomyces cerevisiae.

9. Cellule selon l'une quelconque des revendications précédentes, dans laquelle l'enzyme dépendante de NADP(H) est choisie dans le groupe constitué par les alcool-déshydrogénases, les aldéhyde-déshydrogénases, les lactate-déshydrogénases, les énoate-réductases, les époxyde-réductases, les diaminopimélate-désydrogénases, les acide aminé-déshydrogénases, les aldéhyde-oxydoréductases, les alcane-réductases, les amine-réductases, les époxyde-déshydrogénases, les acide carboxylique-déshydrogénases, les hydroxyacide-cétoréductases et les hydroxyacide-déshalogénases.

10. Procédé d'isolement de gènes, qui codent pour des enzymes dépendantes de NADP(H), comprenant les étapes de procédé suivantes :
(I) la préparation d'un nanocapteur de NADP(H), tel que défini dans l'une quelconque des revendications 1 à 7 ;
(II) l'introduction du nanocapteur de NADP(H) dans une cellule ;
(III) l'introduction d'un gène, qui code éventuellement pour une enzyme dépendante de NADP(H), dans des cellules individuelles d'une suspension cellulaire des cellules obtenues à l'étape de procédé (II) ;
(IV) l'incubation des cellules avec un substrat pour l'enzyme dépendante de NADP(H) ;
(V) l'identification de cellules individuelles dans la suspension cellulaire présentant une activité élevée des enzymes dépendantes de NADP(H) par détection de l'activité de fluorescence intracellulaire ;
(VI) la séparation des cellules identifiées de la suspension cellulaire ;
(VII) l'isolement des gènes codant pour une enzyme dépendante de NADP(H) dans les cellules identifiées.

11. Utilisation d'un nanocapteur de NADP(H), tel que défini dans l'une quelconque des revendications 1 à 7, pour l'identification de gènes, qui codent pour une enzyme dépendante de NADP(H), in vivo.


### SEQUENCE LISTING

<110> Forschungszentrum Jülich GmbH
<120> NADP(H)-Nanosensor
<130> FJ11448
<160> 25
<170> PatentIn version 3.5
<210> 1
   <211> 85
   <212> DNA
   <213> E. coli
<400> 1
   aaatctgcct cttttcagtg ttcagttcgt taattcatct gttggggagt ataattcctc 60
   aagttaactt gaggtaaagc gattt 85
<210> 2
   <211> 465
   <212> DNA
   <213> E. coli
<400> 2
   atggaaaaga aattaccccg cattaaagcg ctgctaaccc ccggcgaagt ggcgaaacgc 60
   agcggtgtgg cggtatcggc gctgcatttc tatgaaagta aagggttgat taccagtatc 120
   cgtaacagcg gcaatcagcg gcgatataaa cgtgatgtgt tgcgatatgt tgcaattatc 180
   aaaattgctc agcgtattgg cattccgctg gcgaccattg gtgaagcgtt tggcgtgttg 240
   cccgaagggc atacgttaag tgcgaaagag tggaaacagc tttcgtccca atggcgagaa 300
   gagttggatc ggcgcattca taccttagtg gcgctgcgtg acgaactgga cggatgtatt 360
   ggttgtggct gcctttcgcg cagtgattgc ccgttgcgta acccgggcga ccgcttagga 420
   gaagaaggta ccggcgcacg cttgctggaa gatgaacaaa actaa 465
<210> 3
   <211> 154
   <212> PRT
   <213> E. coli
<400> 3
   Met Glu Lys Lys Leu Pro Arg Ile Lys Ala Leu Leu Thr Pro Gly Glu
   1               5                   10                  15
   Val Ala Lys Arg Ser Gly Val Ala Val Ser Ala Leu His Phe Tyr Glu
               20                  25                  30
   Ser Lys Gly Leu Ile Thr Ser Ile Arg Asn Ser Gly Asn Gln Arg Arg
           35                  40                  45
   Tyr Lys Arg Asp Val Leu Arg Tyr Val Ala Ile Ile Lys Ile Ala Gln
       50                  55                  60
   Arg Ile Gly Ile Pro Leu Ala Thr Ile Gly Glu Ala Phe Gly Val Leu
   65                  70                  75                  80
   Pro Glu Gly His Thr Leu Ser Ala Lys Glu Trp Lys Gln Leu Ser Ser
                   85                  90                  95
   Gln Trp Arg Glu Glu Leu Asp Arg Arg Ile His Thr Leu Val Ala Leu
               100                 105                 110
   Arg Asp Glu Leu Asp Gly Cys Ile Gly Cys Gly Cys Leu Ser Arg Ser
           115                 120                 125
   Asp Cys Pro Leu Arg Asn Pro Gly Asp Arg Leu Gly Glu Glu Gly Thr
       130                 135                 140
   Gly Ala Arg Leu Leu Glu Asp Glu Gln Asn
   145                 150
<210> 4
   <211> 34
   <212> DNA
   <213> Artificial Sequence
<220>
   <223> SoxS_for_SphI
<400> 4
   atctgcatgc ttacggctgg tcaatatgct cgtc 34
<210> 5
   <211> 30
   <212> DNA
   <213> Artificial Sequence
<220>
   <223> SoxR_rev_SalI
<400> 5
   gctagtcgac caaactaaag cgcccttgtg 30
<210> 6
   <211> 41
   <212> DNA
   <213> Artificial Sequence
<220>
   <223> EYFP_for_SphI
<400> 6
   agaggcatgc aaggagaatt acatggtgag caagggcgag g 41
<210> 7
   <211> 35
   <212> DNA
   <213> Artificial Sequence
<220>
   <223> EYFP rev ClaI
<400> 7
   gcgcatcgat ttattacttg tacagctcgt ccatg 35
<210> 8
   <211> 6436
   <212> DNA
   <213> Artificial Sequenz
<220>
   <223> pSenSox
<400> 8
   ttcatgtcta accgtttgga tggtaaggta gcaatcatta caggtggtac gttgggtatc 60
   ggtttagcta tcgccacgaa gttcgttgaa gaaggggcta aggtcatgat taccggccgg 120
   cacagcgatg ttggtgaaaa agcagctaag agtgtcggca ctcctgatca gattcaattt 180
   ttccaacatg attcttccga tgaagacggc tggacgaaat tattcgatgc aacggaaaaa 240
   gcctttggcc cagtttctac attagttaat aacgctggga tcgcggttaa caagagtgtc 300
   gaagaaacca cgactgctga atggcgtaaa ttattagccg tcaaccttga tggtgtcttc 360
   ttcggtaccc gattagggat tcaacggatg aagaacaaag gcttaggggc ttccatcatc 420
   aacatgtctt cgatcgaagg ctttgtgggt gatcctagct taggggctta caacgcatct 480
   aaaggggccg tacggattat gtccaagtca gctgccttag attgtgccct aaaggactac 540
   gatgttcggg taaacactgt tcaccctggc tacatcaaga caccattggt tgatgaccta 600
   ccaggggccg aagaagcgat gtcacaacgg accaagacgc caatgggcca tatcggtgaa 660
   cctaacgata ttgcctacat ctgtgtttac ttggcttcta acgaatctaa atttgcaacg 720
   ggttctgaat ttgtagttga cggtggctac actgctcaat agtaagcttc tgttttggcg 780
   gatgagagaa gattttcagc ctgatacaga ttaaatcaga acgcagaagc ggtctgataa 840
   aacagaattt gcctggcggc agtagcgcgg tggtcccacc tgaccccatg ccgaactcag 900
   aagtgaaacg ccgtagcgcc gatggtagtg tggggtctcc ccatgcgaga gtagggaact 960
   gccaggcatc aaataaaacg aaaggctcag tcgaaagact gggcctttcg ttttatctgt 1020
   tgtttgtcgg tgaacgctct cctgagtagg acaaatccgc cgggagcgga tttgaacgtt 1080
   gcgaagcaac ggcccggagg gtggcgggca ggacgcccgc cataaactgc caggcatcaa 1140
   attaagcaga aggccatcct gacggatggc ctttttgcgt ttctacaaac tcttttgttt 1200
   atttttctaa atacattcaa atatgtatcc gctcatgaga caataaccct gataaatgct 1260
   tcaataatat tgaaaaagga agagtatgag tattcaacat ttccgtgtcg cccttattcc 1320
   cttttttgcg gcattttgcc ttcctgtttt tgctcaccca gaaacgctgg tgaaagtaaa 1380
   agatgctgaa gatcagttgg gtgcacgagt gggttacatc gaactggatc tcaacagcgg 1440
   taagatcctt gagagttttc gccccgaaga acgttttcca atgatgagca cttttaaagt 1500
   tctgctatgt ggcgcggtat tatcccgtgt tgacgccggg caagagcaac tcggtcgccg 1560
   catacactat tctcagaatg acttggttga gtactcacca gtcacagaaa agcatcttac 1620
   ggatggcatg acagtaagag aattatgcag tgctgccata accatgagtg ataacactgc 1680
   ggccaactta cttctgacaa cgatcggagg accgaaggag ctaaccgctt ttttgcacaa 1740
   catgggggat catgtaactc gccttgatcg ttgggaaccg gagctgaatg aagccatacc 1800
   aaacgacgag cgtgacacca cgatgcctgt agcaatggca acaacgttgc gcaaactatt 1860
   aactggcgaa ctacttactc tagcttcccg gcaacaatta atagactgga tggaggcgga 1920
   taaagttgca ggaccacttc tgcgctcggc ccttccggct ggctggttta ttgctgataa 1980
   atctggagcc ggtgagcgtg ggtctcgcgg tatcattgca gcactggggc cagatggtaa 2040
   gccctcccgt atcgtagtta tctacacgac ggggagtcag gcaactatgg atgaacgaaa 2100
   tagacagatc gctgagatag gtgcctcact gattaagcat tggtaactgt cagaccaagt 2160
   ttactcatat atactttaga ttgatttaaa acttcatttt taatttaaaa ggatctaggt 2220
   gaagatcctt tttgataatc tcatgaccaa aatcccttaa cgtgagtttt cgttccactg 2280
   agcgtcagac cccgtagaaa agatcaaagg atcttcttga gatccttttt ttctgcgcgt 2340
   aatctgctgc ttgcaaacaa aaaaaccacc gctaccagcg gtggtttgtt tgccggatca 2400
   agagctacca actctttttc cgaaggtaac tggcttcagc agagcgcaga taccaaatac 2460
   tgtccttcta gtgtagccgt agttaggcca ccacttcaag aactctgtag caccgcctac 2520
   atacctcgct ctgctaatcc tgttaccagt ggctgctgcc agtggcgata agtcgtgtct 2580
   taccgggttg gactcaagac gatagttacc ggataaggcg cagcggtcgg gctgaacggg 2640
   gggttcgtgc acacagccca gcttggagcg aacgacctac accgaactga gatacctaca 2700
   gcgtgagcta tgagaaagcg ccacgcttcc cgaagggaga aaggcggaca ggtatccggt 2760
   aagcggcagg gtcggaacag gagagcgcac gagggagctt ccagggggaa acgcctggta 2820
   tctttatagt cctgtcgggt ttcgccacct ctgacttgag cgtcgatttt tgtgatgctc 2880
   gtcagggggg cggagcctat ggaaaaacgc cagcaacgcg gcctttttac ggttcctggc 2940
   cttttgctgg ccttttgctc acatgttctt tcctgcgtta tcccctgatt ctgtggataa 3000
   ccgtattacc gcctttgagt gagctgatac cgctcgccgc agccgaacga ccgagcgcag 3060
   cgagtcagtg agcgaggaag cggaagagcg cctgatgcgg tattttctcc ttacgcatct 3120
   gtgcggtatt tcacaccgca tatggtgcac tctcagtaca atctgctctg atgccgcata 3180
   gttaagccag tatacactcc gctatcgcta cgtgactggg tcatggctgc gccccgacac 3240
   ccgccaacac ccgctgacgc gccctgacgg gcttgtctgc tcccggcatc cgcttacaga 3300
   caagctgtga ccgtctccgg gagctgcatg tgtcagaggt tttcaccgtc atcaccgaaa 3360
   cgcgcgaggc agctgcggta aagctcatca gcgtggtcgt gaagcgattc acagatgtct 3420
   gcctgttcat ccgcgtccag ctcgttgagt ttctccagaa gcgttaatgt ctggcttctg 3480
   ataaagcggg ccatgttaag ggcggttttt tcctgtttgg tcacttgatg cctccgtgta 3540
   agggggaatt tctgttcatg ggggtaatga taccgatgaa acgagagagg atgctcacga 3600
   tacgggttac tgatgatgaa catgcccggt tactggaacg ttgtgagggt aaacaactgg 3660
   cggtatggat gcggcgggac cagagaaaaa tcactcaggg tcaatgccag cgcttcgtta 3720
   atacagatgt aggtgttcca cagggtagcc agcagcatcc tgcgatgcag atccggaaca 3780
   taatggtgca gggcgctgac ttccgcgttt ccagacttta cgaaacacgg aaaccgaaga 3840
   ccattcatgt tgttgctcag gtcgcagacg ttttgcagca gcagtcgctt cacgttcgct 3900
   cgcgtatcgg tgattcattc tgctaaccag taaggcaacc ccgccagcct agccgggtcc 3960
   tcaacgacag gagcacgatc atgcgcaccc gtggccagga cccaacgctg cccgagatgc 4020
   gccgcgtgcg gctgctggag atggcggacg cgatggatat gttctgccaa gggttggttt 4080
   gcgcattcac agttctccgc aagaattgat tggctccaat tcttggagtg gtgaatccgt 4140
   tagcgaggtg ccgccggctt ccattcaggt cgaggtggcc cggctccatg caccgcgacg 4200
   caacgcgggg aggcagacaa ggtatagggc ggcgcctaca atccatgcca acccgttcca 4260
   tgtgctcgcc gaggcggcat aaatcgccgt gacgatcagc ggtccagtga tcgaagttag 4320
   gctggtaaga gccgcgagcg atccttgaag ctgtccctga tggtcgtcat ctacctgcct 4380
   ggacagcatg gcctgcaacg cgggcatccc gatgccgccg gaagcgagaa gaatcataat 4440
   ggggaaggcc atccagcctc gcgtcgcgaa cgccagcaag acgtagccca gcgcgtcggc 4500
   cgccatgccg gcgataatgg cctgcttctc gccgaaacgt ttggtggcgg gaccagtgac 4560
   gaaggcttga gcgagggcgt gcaagattcc gaataccgca agcgacaggc cgatcatcgt 4620
   cgcgctccag cgaaagcggt cctcgccgaa aatgacccag agcgctgccg gcacctgtcc 4680
   tacgagttgc atgataaaga agacagtcat aagtgcggcg acgatagtca tgccccgcgc 4740
   ccaccggaag gagctgactg ggttgaaggc tctcaagggc atcggtcgac caaactaaag 4800
   cgcccttgtg gcgctttagt tttgttcatc ttccagcaag cgtgcgccgg taccttcttc 4860
   tcctaagcgg tcgcccgggt tacgcaacgg gcaatcactg cgcgaaaggc agccacaacc 4920
   aatacatccg tccagttcgt cacgcagcgc cactaaggta tgaatgcgcc gatccaactc 4980
   ttctcgccat tgggacgaaa gctgtttcca ctctttcgca cttaacgtat gcccttcggg 5040
   caacacgcca aacgcttcac caatggtcgc cagcggaatg ccaatacgct gagcaatttt 5100
   gataattgca acatatcgca acacatcacg tttatatcgc cgctgattgc cgctgttacg 5160
   gatactggta atcaaccctt tactttcata gaaatgcagc gccgataccg ccacaccgct 5220
   gcgtttcgcc acttcgccgg gggttagcag cgctttaatg cggggtaatt tcttttccat 5280
   aaatcgcttt acctcaagtt aacttgagga attatactcc ccaacagatg aattaacgaa 5340
   ctgaacactg aaaagaggca gatttatgtc ccatcagaaa attattcagg atcttatcgc 5400
   atggattgac gagcatattg accagccgta agcatgcaag gagaattaca tggtgagcaa 5460
   gggcgaggag ctgttcaccg gggtggtgcc catcctggtc gagctggacg gcgacgtaaa 5520
   cggccacaag ttcagcgtgt ccggcgaggg cgagggcgat gccacctacg gcaagctgac 5580
   cctgaagttc atctgcacca ccggcaagct gcccgtgccc tggcccaccc tcgtgaccac 5640
   cttcggctac ggcctgcagt gcttcgcccg ctaccccgac cacatgaagc agcacgactt 5700
   cttcaagtcc gccatgcccg aaggctacgt ccaggagcgc accatcttct tcaaggacga 5760
   cggcaactac aagacccgcg ccgaggtgaa gttcgagggc gacaccctgg tgaaccgcat 5820
   cgagctgaag ggcatcaact tcaaggagga cggcaacatc ctggggcaca agctggagta 5880
   caactacaac agccacaacg tctatatcat ggccgacaag cagaagaacg gcatcaaggt 5940
   gaacttcaag atccgccaca acatcgaggg cggcagcgtg cagctcgccg accactacca 6000
   gcagaacacc cccatcggcg acggccccgt gctgctgccc gacaaccact acctgagcta 6060
   ccagtccgcc ctgagcaaag accccaacga gaagcgcgat cacatggtcc tgctggagtt 6120
   cgtgaccgcc gccgggatca ctctcggcat ggacgagctg tacaagtaat aaatcgatcc 6180
   ggagcttatc gactgcacgg tgcaccaatg cttctggcgt caggcagcca tcggaagctg 6240
   tggtatggct gtgcaggtcg taaatcactg cataattcgt gtcgctcaag gcgcactccc 6300
   gttctggata atgttttttg cgccgacatc ataacggttc tggcaaatat tctgaaatga 6360
   gctgttgaca attaatcatc ggctcgtata atgtgtggaa ttgtgagcgg ataacaattt 6420
   cacacaggaa acagaa 6436
<210> 9
   <211> 30
   <212> DNA
   <213> Artificial Sequence
<220>
   <223> ADH_negK_for
<400> 9
   acaagaattc gctaagagtg tcggcactcc 30
<210> 10
   <211> 30
   <212> DNA
   <213> Artificial Sequence
<220>
   <223> ADH_negK_rev
<400> 10
   ggccaagctt ccgaagaaga caccatcaag 30
<210> 11
   <211> 5897
   <212> DNA
   <213> Artificial Sequence
<220>
   <223> vector
<400> 11
   agcttctgtt ttggcggatg agagaagatt ttcagcctga tacagattaa atcagaacgc 60
   agaagcggtc tgataaaaca gaatttgcct ggcggcagta gcgcggtggt cccacctgac 120
   cccatgccga actcagaagt gaaacgccgt agcgccgatg gtagtgtggg gtctccccat 180
   gcgagagtag ggaactgcca ggcatcaaat aaaacgaaag gctcagtcga aagactgggc 240
   ctttcgtttt atctgttgtt tgtcggtgaa cgctctcctg agtaggacaa atccgccggg 300
   agcggatttg aacgttgcga agcaacggcc cggagggtgg cgggcaggac gcccgccata 360
   aactgccagg catcaaatta agcagaaggc catcctgacg gatggccttt ttgcgtttct 420
   acaaactctt ttgtttattt ttctaaatac attcaaatat gtatccgctc atgagacaat 480
   aaccctgata aatgcttcaa taatattgaa aaaggaagag tatgagtatt caacatttcc 540
   gtgtcgccct tattcccttt tttgcggcat tttgccttcc tgtttttgct cacccagaaa 600
   cgctggtgaa agtaaaagat gctgaagatc agttgggtgc acgagtgggt tacatcgaac 660
   tggatctcaa cagcggtaag atccttgaga gttttcgccc cgaagaacgt tttccaatga 720
   tgagcacttt taaagttctg ctatgtggcg cggtattatc ccgtgttgac gccgggcaag 780
   agcaactcgg tcgccgcata cactattctc agaatgactt ggttgagtac tcaccagtca 840
   cagaaaagca tcttacggat ggcatgacag taagagaatt atgcagtgct gccataacca 900
   tgagtgataa cactgcggcc aacttacttc tgacaacgat cggaggaccg aaggagctaa 960
   ccgctttttt gcacaacatg ggggatcatg taactcgcct tgatcgttgg gaaccggagc 1020
   tgaatgaagc cataccaaac gacgagcgtg acaccacgat gcctgtagca atggcaacaa 1080
   cgttgcgcaa actattaact ggcgaactac ttactctagc ttcccggcaa caattaatag 1140
   actggatgga ggcggataaa gttgcaggac cacttctgcg ctcggccctt ccggctggct 1200
   ggtttattgc tgataaatct ggagccggtg agcgtgggtc tcgcggtatc attgcagcac 1260
   tggggccaga tggtaagccc tcccgtatcg tagttatcta cacgacgggg agtcaggcaa 1320
   ctatggatga acgaaataga cagatcgctg agataggtgc ctcactgatt aagcattggt 1380
   aactgtcaga ccaagtttac tcatatatac tttagattga tttaaaactt catttttaat 1440
   ttaaaaggat ctaggtgaag atcctttttg ataatctcat gaccaaaatc ccttaacgtg 1500
   agttttcgtt ccactgagcg tcagaccccg tagaaaagat caaaggatct tcttgagatc 1560
   ctttttttct gcgcgtaatc tgctgcttgc aaacaaaaaa accaccgcta ccagcggtgg 1620
   tttgtttgcc ggatcaagag ctaccaactc tttttccgaa ggtaactggc ttcagcagag 1680
   cgcagatacc aaatactgtc cttctagtgt agccgtagtt aggccaccac ttcaagaact 1740
   ctgtagcacc gcctacatac ctcgctctgc taatcctgtt accagtggct gctgccagtg 1800
   gcgataagtc gtgtcttacc gggttggact caagacgata gttaccggat aaggcgcagc 1860
   ggtcgggctg aacggggggt tcgtgcacac agcccagctt ggagcgaacg acctacaccg 1920
   aactgagata cctacagcgt gagctatgag aaagcgccac gcttcccgaa gggagaaagg 1980
   cggacaggta tccggtaagc ggcagggtcg gaacaggaga gcgcacgagg gagcttccag 2040
   ggggaaacgc ctggtatctt tatagtcctg tcgggtttcg ccacctctga cttgagcgtc 2100
   gatttttgtg atgctcgtca ggggggcgga gcctatggaa aaacgccagc aacgcggcct 2160
   ttttacggtt cctggccttt tgctggcctt ttgctcacat gttctttcct gcgttatccc 2220
   ctgattctgt ggataaccgt attaccgcct ttgagtgagc tgataccgct cgccgcagcc 2280
   gaacgaccga gcgcagcgag tcagtgagcg aggaagcgga agagcgcctg atgcggtatt 2340
   ttctccttac gcatctgtgc ggtatttcac accgcatatg gtgcactctc agtacaatct 2400
   gctctgatgc cgcatagtta agccagtata cactccgcta tcgctacgtg actgggtcat 2460
   ggctgcgccc cgacacccgc caacacccgc tgacgcgccc tgacgggctt gtctgctccc 2520
   ggcatccgct tacagacaag ctgtgaccgt ctccgggagc tgcatgtgtc agaggttttc 2580
   accgtcatca ccgaaacgcg cgaggcagct gcggtaaagc tcatcagcgt ggtcgtgaag 2640
   cgattcacag atgtctgcct gttcatccgc gtccagctcg ttgagtttct ccagaagcgt 2700
   taatgtctgg cttctgataa agcgggccat gttaagggcg gttttttcct gtttggtcac 2760
   ttgatgcctc cgtgtaaggg ggaatttctg ttcatggggg taatgatacc gatgaaacga 2820
   gagaggatgc tcacgatacg ggttactgat gatgaacatg cccggttact ggaacgttgt 2880
   gagggtaaac aactggcggt atggatgcgg cgggaccaga gaaaaatcac tcagggtcaa 2940
   tgccagcgct tcgttaatac agatgtaggt gttccacagg gtagccagca gcatcctgcg 3000
   atgcagatcc ggaacataat ggtgcagggc gctgacttcc gcgtttccag actttacgaa 3060
   acacggaaac cgaagaccat tcatgttgtt gctcaggtcg cagacgtttt gcagcagcag 3120
   tcgcttcacg ttcgctcgcg tatcggtgat tcattctgct aaccagtaag gcaaccccgc 3180
   cagcctagcc gggtcctcaa cgacaggagc acgatcatgc gcacccgtgg ccaggaccca 3240
   acgctgcccg agatgcgccg cgtgcggctg ctggagatgg cggacgcgat ggatatgttc 3300
   tgccaagggt tggtttgcgc attcacagtt ctccgcaaga attgattggc tccaattctt 3360
   ggagtggtga atccgttagc gaggtgccgc cggcttccat tcaggtcgag gtggcccggc 3420
   tccatgcacc gcgacgcaac gcggggaggc agacaaggta tagggcggcg cctacaatcc 3480
   atgccaaccc gttccatgtg ctcgccgagg cggcataaat cgccgtgacg atcagcggtc 3540
   cagtgatcga agttaggctg gtaagagccg cgagcgatcc ttgaagctgt ccctgatggt 3600
   cgtcatctac ctgcctggac agcatggcct gcaacgcggg catcccgatg ccgccggaag 3660
   cgagaagaat cataatgggg aaggccatcc agcctcgcgt cgcgaacgcc agcaagacgt 3720
   agcccagcgc gtcggccgcc atgccggcga taatggcctg cttctcgccg aaacgtttgg 3780
   tggcgggacc agtgacgaag gcttgagcga gggcgtgcaa gattccgaat accgcaagcg 3840
   acaggccgat catcgtcgcg ctccagcgaa agcggtcctc gccgaaaatg acccagagcg 3900
   ctgccggcac ctgtcctacg agttgcatga taaagaagac agtcataagt gcggcgacga 3960
   tagtcatgcc ccgcgcccac cggaaggagc tgactgggtt gaaggctctc aagggcatcg 4020
   gtcgaccaaa ctaaagcgcc cttgtggcgc tttagttttg ttcatcttcc agcaagcgtg 4080
   cgccggtacc ttcttctcct aagcggtcgc ccgggttacg caacgggcaa tcactgcgcg 4140
   aaaggcagcc acaaccaata catccgtcca gttcgtcacg cagcgccact aaggtatgaa 4200
   tgcgccgatc caactcttct cgccattggg acgaaagctg tttccactct ttcgcactta 4260
   acgtatgccc ttcgggcaac acgccaaacg cttcaccaat ggtcgccagc ggaatgccaa 4320
   tacgctgagc aattttgata attgcaacat atcgcaacac atcacgttta tatcgccgct 4380
   gattgccgct gttacggata ctggtaatca accctttact ttcatagaaa tgcagcgccg 4440
   ataccgccac accgctgcgt ttcgccactt cgccgggggt tagcagcgct ttaatgcggg 4500
   gtaatttctt ttccataaat cgctttacct caagttaact tgaggaatta tactccccaa 4560
   cagatgaatt aacgaactga acactgaaaa gaggcagatt tatgtcccat cagaaaatta 4620
   ttcaggatct tatcgcatgg attgacgagc atattgacca gccgtaagca tgcaaggaga 4680
   attacatggt gagcaagggc gaggagctgt tcaccggggt ggtgcccatc ctggtcgagc 4740
   tggacggcga cgtaaacggc cacaagttca gcgtgtccgg cgagggcgag ggcgatgcca 4800
   cctacggcaa gctgaccctg aagttcatct gcaccaccgg caagctgccc gtgccctggc 4860
   ccaccctcgt gaccaccttc ggctacggcc tgcagtgctt cgcccgctac cccgaccaca 4920
   tgaagcagca cgacttcttc aagtccgcca tgcccgaagg ctacgtccag gagcgcacca 4980
   tcttcttcaa ggacgacggc aactacaaga cccgcgccga ggtgaagttc gagggcgaca 5040
   ccctggtgaa ccgcatcgag ctgaagggca tcaacttcaa ggaggacggc aacatcctgg     5100
   ggcacaagct ggagtacaac tacaacagcc acaacgtcta tatcatggcc gacaagcaga     5160
   agaacggcat caaggtgaac ttcaagatcc gccacaacat cgagggcggc agcgtgcagc     5220
   tcgccgacca ctaccagcag aacaccccca tcggcgacgg ccccgtgctg ctgcccgaca     5280
   accactacct gagctaccag tccgccctga gcaaagaccc caacgagaag cgcgatcaca     5340
   tggtcctgct ggagttcgtg accgccgccg ggatcactct cggcatggac gagctgtaca     5400
   agtaataaat cgatccggag cttatcgact gcacggtgca ccaatgcttc tggcgtcagg     5460
   cagccatcgg aagctgtggt atggctgtgc aggtcgtaaa tcactgcata attcgtgtcg     5520
   ctcaaggcgc actcccgttc tggataatgt tttttgcgcc gacatcataa cggttctggc     5580
   aaatattctg aaatgagctg ttgacaatta atcatcggct cgtataatgt gtggaattgt     5640
   gagcggataa caatttcaca caggaaacag aattcgctaa gagtgtcggc actcctgatc     5700
   agattcaatt tttccaacat gattcttccg atgaagacgg ctggacgaaa ttattcgatg     5760
   caacggaaaa agcctttggc ccagtttcta cattagttaa taacgctggg atcgcggtta     5820
   acaagagtgt cgaagaaacc acgactgctg aatggcgtaa attattagcc gtcaaccttg     5880
   atggtgtctt cttcgga                                                    5897
<210> 12
   <211> 30
   <212> DNA
   <213> Artificial Sequence
<220>
   <223> L194S_for
<400> 12
   ctggctacat caagacacca tctgttgatg 30
<210> 13
   <211> 30
   <212> DNA
   <213> Artificial Sequence
<220>
   <223> L194S_rev
<400> 13
   cggcccctgg taggtcatca acagatggtg 30
<210> 14
   <211> 6436
   <212> DNA
   <213> Artificial Sequence
<220>
   <223> vector
<400> 14
   ttcatgtcta accgtttgga tggtaaggta gcaatcatta caggtggtac gttgggtatc      60
   ggtttagcta tcgccacgaa gttcgttgaa gaaggggcta aggtcatgat taccggccgg     120
   cacagcgatg ttggtgaaaa agcagctaag agtgtcggca ctcctgatca gattcaattt     180
   ttccaacatg attcttccga tgaagacggc tggacgaaat tattcgatgc aacggaaaaa     240
   gcctttggcc cagtttctac attagttaat aacgctggga tcgcggttaa caagagtgtc     300
   gaagaaacca cgactgctga atggcgtaaa ttattagccg tcaaccttga tggtgtcttc     360
   ttcggtaccc gattagggat tcaacggatg aagaacaaag gcttaggggc ttccatcatc     420
   aacatgtctt cgatcgaagg ctttgtgggt gatcctagct taggggctta caacgcatct     480
   aaaggggccg tacggattat gtccaagtca gctgccttag attgtgccct aaaggactac     540
   gatgttcggg taaacactgt tcaccctggc tacatcaaga caccatctgt tgatgaccta     600
   ccaggggccg aagaagcgat gtcacaacgg accaagacgc caatgggcca tatcggtgaa     660
   cctaacgata ttgcctacat ctgtgtttac ttggcttcta acgaatctaa atttgcaacg     720
   ggttctgaat ttgtagttga cggtggctac actgctcaat agtaagcttc tgttttggcg     780
   gatgagagaa gattttcagc ctgatacaga ttaaatcaga acgcagaagc ggtctgataa     840
   aacagaattt gcctggcggc agtagcgcgg tggtcccacc tgaccccatg ccgaactcag     900
   aagtgaaacg ccgtagcgcc gatggtagtg tggggtctcc ccatgcgaga gtagggaact     960
   gccaggcatc aaataaaacg aaaggctcag tcgaaagact gggcctttcg ttttatctgt    1020
   tgtttgtcgg tgaacgctct cctgagtagg acaaatccgc cgggagcgga tttgaacgtt    1080
   gcgaagcaac ggcccggagg gtggcgggca ggacgcccgc cataaactgc caggcatcaa    1140
   attaagcaga aggccatcct gacggatggc ctttttgcgt ttctacaaac tcttttgttt    1200
   atttttctaa atacattcaa atatgtatcc gctcatgaga caataaccct gataaatgct    1260
   tcaataatat tgaaaaagga agagtatgag tattcaacat ttccgtgtcg cccttattcc    1320
   cttttttgcg gcattttgcc ttcctgtttt tgctcaccca gaaacgctgg tgaaagtaaa    1380
   agatgctgaa gatcagttgg gtgcacgagt gggttacatc gaactggatc tcaacagcgg    1440
   taagatcctt gagagttttc gccccgaaga acgttttcca atgatgagca cttttaaagt    1500
   tctgctatgt ggcgcggtat tatcccgtgt tgacgccggg caagagcaac tcggtcgccg    1560
   catacactat tctcagaatg acttggttga gtactcacca gtcacagaaa agcatcttac    1620
   ggatggcatg acagtaagag aattatgcag tgctgccata accatgagtg ataacactgc    1680
   ggccaactta cttctgacaa cgatcggagg accgaaggag ctaaccgctt ttttgcacaa    1740
   catgggggat catgtaactc gccttgatcg ttgggaaccg gagctgaatg aagccatacc    1800
   aaacgacgag cgtgacacca cgatgcctgt agcaatggca acaacgttgc gcaaactatt    1860
   aactggcgaa ctacttactc tagcttcccg gcaacaatta atagactgga tggaggcgga 1920
   taaagttgca ggaccacttc tgcgctcggc ccttccggct ggctggttta ttgctgataa 1980
   atctggagcc ggtgagcgtg ggtctcgcgg tatcattgca gcactggggc cagatggtaa 2040
   gccctcccgt atcgtagtta tctacacgac ggggagtcag gcaactatgg atgaacgaaa 2100
   tagacagatc gctgagatag gtgcctcact gattaagcat tggtaactgt cagaccaagt 2160
   ttactcatat atactttaga ttgatttaaa acttcatttt taatttaaaa ggatctaggt 2220
   gaagatcctt tttgataatc tcatgaccaa aatcccttaa cgtgagtttt cgttccactg 2280
   agcgtcagac cccgtagaaa agatcaaagg atcttcttga gatccttttt ttctgcgcgt 2340
   aatctgctgc ttgcaaacaa aaaaaccacc gctaccagcg gtggtttgtt tgccggatca 2400
   agagctacca actctttttc cgaaggtaac tggcttcagc agagcgcaga taccaaatac 2460
   tgtccttcta gtgtagccgt agttaggcca ccacttcaag aactctgtag caccgcctac 2520
   atacctcgct ctgctaatcc tgttaccagt ggctgctgcc agtggcgata agtcgtgtct 2580
   taccgggttg gactcaagac gatagttacc ggataaggcg cagcggtcgg gctgaacggg 2640
   gggttcgtgc acacagccca gcttggagcg aacgacctac accgaactga gatacctaca 2700
   gcgtgagcta tgagaaagcg ccacgcttcc cgaagggaga aaggcggaca ggtatccggt 2760
   aagcggcagg gtcggaacag gagagcgcac gagggagctt ccagggggaa acgcctggta 2820
   tctttatagt cctgtcgggt ttcgccacct ctgacttgag cgtcgatttt tgtgatgctc 2880
   gtcagggggg cggagcctat ggaaaaacgc cagcaacgcg gcctttttac ggttcctggc 2940
   cttttgctgg ccttttgctc acatgttctt tcctgcgtta tcccctgatt ctgtggataa 3000
   ccgtattacc gcctttgagt gagctgatac cgctcgccgc agccgaacga ccgagcgcag 3060
   cgagtcagtg agcgaggaag cggaagagcg cctgatgcgg tattttctcc ttacgcatct 3120
   gtgcggtatt tcacaccgca tatggtgcac tctcagtaca atctgctctg atgccgcata 3180
   gttaagccag tatacactcc gctatcgcta cgtgactggg tcatggctgc gccccgacac 3240
   ccgccaacac ccgctgacgc gccctgacgg gcttgtctgc tcccggcatc cgcttacaga 3300
   caagctgtga ccgtctccgg gagctgcatg tgtcagaggt tttcaccgtc atcaccgaaa 3360
   cgcgcgaggc agctgcggta aagctcatca gcgtggtcgt gaagcgattc acagatgtct 3420
   gcctgttcat ccgcgtccag ctcgttgagt ttctccagaa gcgttaatgt ctggcttctg 3480
   ataaagcggg ccatgttaag ggcggttttt tcctgtttgg tcacttgatg cctccgtgta 3540
   agggggaatt tctgttcatg ggggtaatga taccgatgaa acgagagagg atgctcacga 3600
   tacgggttac tgatgatgaa catgcccggt tactggaacg ttgtgagggt aaacaactgg 3660
   cggtatggat gcggcgggac cagagaaaaa tcactcaggg tcaatgccag cgcttcgtta 3720
   atacagatgt aggtgttcca cagggtagcc agcagcatcc tgcgatgcag atccggaaca 3780
   taatggtgca gggcgctgac ttccgcgttt ccagacttta cgaaacacgg aaaccgaaga 3840
   ccattcatgt tgttgctcag gtcgcagacg ttttgcagca gcagtcgctt cacgttcgct 3900
   cgcgtatcgg tgattcattc tgctaaccag taaggcaacc ccgccagcct agccgggtcc 3960
   tcaacgacag gagcacgatc atgcgcaccc gtggccagga cccaacgctg cccgagatgc 4020
   gccgcgtgcg gctgctggag atggcggacg cgatggatat gttctgccaa gggttggttt 4080
   gcgcattcac agttctccgc aagaattgat tggctccaat tcttggagtg gtgaatccgt 4140
   tagcgaggtg ccgccggctt ccattcaggt cgaggtggcc cggctccatg caccgcgacg 4200
   caacgcgggg aggcagacaa ggtatagggc ggcgcctaca atccatgcca acccgttcca 4260
   tgtgctcgcc gaggcggcat aaatcgccgt gacgatcagc ggtccagtga tcgaagttag 4320
   gctggtaaga gccgcgagcg atccttgaag ctgtccctga tggtcgtcat ctacctgcct 4380
   ggacagcatg gcctgcaacg cgggcatccc gatgccgccg gaagcgagaa gaatcataat 4440
   ggggaaggcc atccagcctc gcgtcgcgaa cgccagcaag acgtagccca gcgcgtcggc 4500
   cgccatgccg gcgataatgg cctgcttctc gccgaaacgt ttggtggcgg gaccagtgac 4560
   gaaggcttga gcgagggcgt gcaagattcc gaataccgca agcgacaggc cgatcatcgt 4620
   cgcgctccag cgaaagcggt cctcgccgaa aatgacccag agcgctgccg gcacctgtcc 4680
   tacgagttgc atgataaaga agacagtcat aagtgcggcg acgatagtca tgccccgcgc 4740
   ccaccggaag gagctgactg ggttgaaggc tctcaagggc atcggtcgac caaactaaag 4800
   cgcccttgtg gcgctttagt tttgttcatc ttccagcaag cgtgcgccgg taccttcttc 4860
   tcctaagcgg tcgcccgggt tacgcaacgg gcaatcactg cgcgaaaggc agccacaacc 4920
   aatacatccg tccagttcgt cacgcagcgc cactaaggta tgaatgcgcc gatccaactc 4980
   ttctcgccat tgggacgaaa gctgtttcca ctctttcgca cttaacgtat gcccttcggg 5040
   caacacgcca aacgcttcac caatggtcgc cagcggaatg ccaatacgct gagcaatttt 5100
   gataattgca acatatcgca acacatcacg tttatatcgc cgctgattgc cgctgttacg 5160
   gatactggta atcaaccctt tactttcata gaaatgcagc gccgataccg ccacaccgct 5220
   gcgtttcgcc acttcgccgg gggttagcag cgctttaatg cggggtaatt tcttttccat 5280
   aaatcgcttt acctcaagtt aacttgagga attatactcc ccaacagatg aattaacgaa 5340
   ctgaacactg aaaagaggca gatttatgtc ccatcagaaa attattcagg atcttatcgc 5400
   atggattgac gagcatattg accagccgta agcatgcaag gagaattaca tggtgagcaa 5460
   gggcgaggag ctgttcaccg ggtggtgccc catcctggtc gagctggacg gcgacgtaaa 5520
   cggccacaag ttcagcgtgt ccggcgaggg cgagggcgat gccacctacg gcaagctgac 5580
   cctgaagttc atctgcacca ccggcaagct gcccgtgccc tggcccaccc tcgtgaccac 5640
   cttcggctac ggcctgcagt gcttcgcccg ctaccccgac cacatgaagc agcacgactt 5700
   cttcaagtcc gccatgcccg aaggctacgt ccaggagcgc accatcttct tcaaggacga 5760
   cggcaactac aagacccgcg ccgaggtgaa gttcgagggc gacaccctgg tgaaccgcat 5820
   cgagctgaag ggcatcaact tcaaggagga cggcaacatc ctggggcaca agctggagta 5880
   caactacaac agccacaacg tctatatcat ggccgacaag cagaagaacg gcatcaaggt 5940
   gaacttcaag atccgccaca acatcgaggg cggcagcgtg cagctcgccg accactacca 6000
   gcagaacacc cccatcggcg acggccccgt gctgctgccc gacaaccact acctgagcta 6060
   ccagtccgcc ctgagcaaag accccaacga gaagcgcgat cacatggtcc tgctggagtt 6120
   cgtgaccgcc gccgggatca ctctcggcat ggacgagctg tacaagtaat aaatcgatcc 6180
   ggagcttatc gactgcacgg tgcaccaatg cttctggcgt caggcagcca tcggaagctg 6240
   tggtatggct gtgcaggtcg taaatcactg cataattcgt gtcgctcaag gcgcactccc 6300
   gttctggata atgttttttg cgccgacatc ataacggttc tggcaaatat tctgaaatga 6360
   gctgttgaca attaatcatc ggctcgtata atgtgtggaa ttgtgagcgg ataacaattt 6420
   cacacaggaa acagaa 6436
<210> 15
   <211> 30
   <212> DNA
   <213> Artificial Sequence
<220>
   <223> L194A_for
<400> 15
   ctggctacat caagacacca gcggttgatg 30
<210> 16
   <211> 30
   <212> DNA
   <213> Artificial Sequence
<220>
   <223> L194A_rev
<400> 16
   cggcccctgg taggtcatca accgctggtg 30
<210> 17
   <211> 6436
   <212> DNA
   <213> Artificial Sequence
<220>
   <223> vector
<400> 17
   ttcatgtcta accgtttgga tggtaaggta gcaatcatta caggtggtac gttgggtatc 60
   ggtttagcta tcgccacgaa gttcgttgaa gaaggggcta aggtcatgat taccggccgg 120
   cacagcgatg ttggtgaaaa agcagctaag agtgtcggca ctcctgatca gattcaattt 180
   ttccaacatg attcttccga tgaagacggc tggacgaaat tattcgatgc aacggaaaaa 240
   gcctttggcc cagtttctac attagttaat aacgctggga tcgcggttaa caagagtgtc 300
   gaagaaacca cgactgctga atggcgtaaa ttattagccg tcaaccttga tggtgtcttc 360
   ttcggtaccc gattagggat tcaacggatg aagaacaaag gcttaggggc ttccatcatc 420
   aacatgtctt cgatcgaagg ctttgtgggt gatcctagct taggggctta caacgcatct 480
   aaaggggccg tacggattat gtccaagtca gctgccttag attgtgccct aaaggactac 540
   gatgttcggg taaacactgt tcaccctggc tacatcaaga caccagcggt tgatgaccta 600
   ccaggggccg aagaagcgat gtcacaacgg accaagacgc caatgggcca tatcggtgaa 660
   cctaacgata ttgcctacat ctgtgtttac ttggcttcta acgaatctaa atttgcaacg 720
   ggttctgaat ttgtagttga cggtggctac actgctcaat agtaagcttc tgttttggcg 780
   gatgagagaa gattttcagc ctgatacaga ttaaatcaga acgcagaagc ggtctgataa 840
   aacagaattt gcctggcggc agtagcgcgg tggtcccacc tgaccccatg ccgaactcag 900
   aagtgaaacg ccgtagcgcc gatggtagtg tggggtctcc ccatgcgaga gtagggaact 960
   gccaggcatc aaataaaacg aaaggctcag tcgaaagact gggcctttcg ttttatctgt 1020
   tgtttgtcgg tgaacgctct cctgagtagg acaaatccgc cgggagcgga tttgaacgtt 1080
   gcgaagcaac ggcccggagg gtggcgggca ggacgcccgc cataaactgc caggcatcaa 1140
   attaagcaga aggccatcct gacggatggc ctttttgcgt ttctacaaac tcttttgttt 1200
   atttttctaa atacattcaa atatgtatcc gctcatgaga caataaccct gataaatgct 1260
   tcaataatat tgaaaaagga agagtatgag tattcaacat ttccgtgtcg cccttattcc 1320
   cttttttgcg gcattttgcc ttcctgtttt tgctcaccca gaaacgctgg tgaaagtaaa 1380
   agatgctgaa gatcagttgg gtgcacgagt gggttacatc gaactggatc tcaacagcgg 1440
   taagatcctt gagagttttc gccccgaaga acgttttcca atgatgagca cttttaaagt 1500
   tctgctatgt ggcgcggtat tatcccgtgt tgacgccggg caagagcaac tcggtcgccg 1560
   catacactat tctcagaatg acttggttga gtactcacca gtcacagaaa agcatcttac 1620
   ggatggcatg acagtaagag aattatgcag tgctgccata accatgagtg ataacactgc 1680
   ggccaactta cttctgacaa cgatcggagg accgaaggag ctaaccgctt ttttgcacaa 1740
   catgggggat catgtaactc gccttgatcg ttgggaaccg gagctgaatg aagccatacc 1800
   aaacgacgag cgtgacacca cgatgcctgt agcaatggca acaacgttgc gcaaactatt 1860
   aactggcgaa ctacttactc tagcttcccg gcaacaatta atagactgga tggaggcgga 1920
   taaagttgca ggaccacttc tgcgctcggc ccttccggct ggctggttta ttgctgataa 1980
   atctggagcc ggtgagcgtg ggtctcgcgg tatcattgca gcactggggc cagatggtaa 2040
   gccctcccgt atcgtagtta tctacacgac ggggagtcag gcaactatgg atgaacgaaa 2100
   tagacagatc gctgagatag gtgcctcact gattaagcat tggtaactgt cagaccaagt 2160
   ttactcatat atactttaga ttgatttaaa acttcatttt taatttaaaa ggatctaggt 2220
   gaagatcctt tttgataatc tcatgaccaa aatcccttaa cgtgagtttt cgttccactg 2280
   agcgtcagac cccgtagaaa agatcaaagg atcttcttga gatccttttt ttctgcgcgt 2340
   aatctgctgc ttgcaaacaa aaaaaccacc gctaccagcg gtggtttgtt tgccggatca 2400
   agagctacca actctttttc cgaaggtaac tggcttcagc agagcgcaga taccaaatac 2460
   tgtccttcta gtgtagccgt agttaggcca ccacttcaag aactctgtag caccgcctac 2520
   atacctcgct ctgctaatcc tgttaccagt ggctgctgcc agtggcgata agtcgtgtct 2580
   taccgggttg gactcaagac gatagttacc ggataaggcg cagcggtcgg gctgaacggg 2640
   gggttcgtgc acacagccca gcttggagcg aacgacctac accgaactga gatacctaca 2700
   gcgtgagcta tgagaaagcg ccacgcttcc cgaagggaga aaggcggaca ggtatccggt 2760
   aagcggcagg gtcggaacag gagagcgcac gagggagctt ccagggggaa acgcctggta 2820
   tctttatagt cctgtcgggt ttcgccacct ctgacttgag cgtcgatttt tgtgatgctc 2880
   gtcagggggg cggagcctat ggaaaaacgc cagcaacgcg gcctttttac ggttcctggc 2940
   cttttgctgg ccttttgctc acatgttctt tcctgcgtta tcccctgatt ctgtggataa 3000
   ccgtattacc gcctttgagt gagctgatac cgctcgccgc agccgaacga ccgagcgcag 3060
   cgagtcagtg agcgaggaag cggaagagcg cctgatgcgg tattttctcc ttacgcatct 3120
   gtgcggtatt tcacaccgca tatggtgcac tctcagtaca atctgctctg atgccgcata 3180
   gttaagccag tatacactcc gctatcgcta cgtgactggg tcatggctgc gccccgacac 3240
   ccgccaacac ccgctgacgc gccctgacgg gcttgtctgc tcccggcatc cgcttacaga 3300
   caagctgtga ccgtctccgg gagctgcatg tgtcagaggt tttcaccgtc atcaccgaaa 3360
   cgcgcgaggc agctgcggta aagctcatca gcgtggtcgt gaagcgattc acagatgtct 3420
   gcctgttcat ccgcgtccag ctcgttgagt ttctccagaa gcgttaatgt ctggcttctg 3480
   ataaagcggg ccatgttaag ggcggttttt tcctgtttgg tcacttgatg cctccgtgta 3540
   agggggaatt tctgttcatg ggggtaatga taccgatgaa acgagagagg atgctcacga 3600
   tacgggttac tgatgatgaa catgcccggt tactggaacg ttgtgagggt aaacaactgg 3660
   cggtatggat gcggcgggac cagagaaaaa tcactcaggg tcaatgccag cgcttcgtta 3720
   atacagatgt aggtgttcca cagggtagcc agcagcatcc tgcgatgcag atccggaaca 3780
   taatggtgca gggcgctgac ttccgcgttt ccagacttta cgaaacacgg aaaccgaaga 3840
   ccattcatgt tgttgctcag gtcgcagacg ttttgcagca gcagtcgctt cacgttcgct 3900
   cgcgtatcgg tgattcattc tgctaaccag taaggcaacc ccgccagcct agccgggtcc 3960
   tcaacgacag gagcacgatc atgcgcaccc gtggccagga cccaacgctg cccgagatgc 4020
   gccgcgtgcg gctgctggag atggcggacg cgatggatat gttctgccaa gggttggttt 4080
   gcgcattcac agttctccgc aagaattgat tggctccaat tcttggagtg gtgaatccgt 4140
   tagcgaggtg ccgccggctt ccattcaggt cgaggtggcc cggctccatg caccgcgacg 4200
   caacgcgggg aggcagacaa ggtatagggc ggcgcctaca atccatgcca acccgttcca 4260
   tgtgctcgcc gaggcggcat aaatcgccgt gacgatcagc ggtccagtga tcgaagttag 4320
   gctggtaaga gccgcgagcg atccttgaag ctgtccctga tggtcgtcat ctacctgcct 4380
   ggacagcatg gcctgcaacg cgggcatccc gatgccgccg gaagcgagaa gaatcataat 4440
   ggggaaggcc atccagcctc gcgtcgcgaa cgccagcaag acgtagccca gcgcgtcggc 4500
   cgccatgccg gcgataatgg cctgcttctc gccgaaacgt ttggtggcgg gaccagtgac 4560
   gaaggcttga gcgagggcgt gcaagattcc gaataccgca agcgacaggc cgatcatcgt 4620
   cgcgctccag cgaaagcggt cctcgccgaa aatgacccag agcgctgccg gcacctgtcc 4680
   tacgagttgc atgataaaga agacagtcat aagtgcggcg acgatagtca tgccccgcgc 4740
   ccaccggaag gagctgactg ggttgaaggc tctcaagggc atcggtcgac caaactaaag 4800
   cgcccttgtg gcgctttagt tttgttcatc ttccagcaag cgtgcgccgg taccttcttc 4860
   tcctaagcgg tcgcccgggt tacgcaacgg gcaatcactg cgcgaaaggc agccacaacc 4920
   aatacatccg tccagttcgt cacgcagcgc cactaaggta tgaatgcgcc gatccaactc 4980
   ttctcgccat tgggacgaaa gctgtttcca ctctttcgca cttaacgtat gcccttcggg 5040
   caacacgcca aacgcttcac caatggtcgc cagcggaatg ccaatacgct gagcaatttt 5100
   gataattgca acatatcgca acacatcacg tttatatcgc cgctgattgc cgctgttacg 5160
   gatactggta atcaaccctt tactttcata gaaatgcagc gccgataccg ccacaccgct 5220
   gcgtttcgcc acttcgccgg gggttagcag cgctttaatg cggggtaatt tcttttccat 5280
   aaatcgcttt acctcaagtt aacttgagga attatactcc ccaacagatg aattaacgaa 5340
   ctgaacactg aaaagaggca gatttatgtc ccatcagaaa attattcagg atcttatcgc 5400
   atggattgac gagcatattg accagccgta agcatgcaag gagaattaca tggtgagcaa 5460
   gggcgaggag ctgttcaccg ggtggtgccc catcctggtc gagctggacg gcgacgtaaa 5520
   cggccacaag ttcagcgtgt ccggcgaggg cgagggcgat gccacctacg gcaagctgac 5580
   cctgaagttc atctgcacca ccggcaagct gcccgtgccc tggcccaccc tcgtgaccac 5640
   cttcggctac ggcctgcagt gcttcgcccg ctaccccgac cacatgaagc agcacgactt 5700
   cttcaagtcc gccatgcccg aaggctacgt ccaggagcgc accatcttct tcaaggacga      5760
   cggcaactac aagacccgcg ccgaggtgaa gttcgagggc gacaccctgg tgaaccgcat      5820
   cgagctgaag ggcatcaact tcaaggagga cggcaacatc ctggggcaca agctggagta      5880
   caactacaac agccacaacg tctatatcat ggccgacaag cagaagaacg gcatcaaggt      5940
   gaacttcaag atccgccaca acatcgaggg cggcagcgtg cagctcgccg accactacca      6000
   gcagaacacc cccatcggcg acggccccgt gctgctgccc gacaaccact acctgagcta      6060
   ccagtccgcc ctgagcaaag accccaacga gaagcgcgat cacatggtcc tgctggagtt      6120
   cgtgaccgcc gccgggatca ctctcggcat ggacgagctg tacaagtaat aaatcgatcc      6180
   ggagcttatc gactgcacgg tgcaccaatg cttctggcgt caggcagcca tcggaagctg      6240
   tggtatggct gtgcaggtcg taaatcactg cataattcgt gtcgctcaag gcgcactccc      6300
   gttctggata atgttttttg cgccgacatc ataacggttc tggcaaatat tctgaaatga      6360
   gctgttgaca attaatcatc ggctcgtata atgtgtggaa ttgtgagcgg ataacaattt      6420
   cacacaggaa acagaa                                                       6436
<210> 18
   <211> 30
   <212> DNA
   <213> Artificial Sequence
<220>
   <223> primer
<400> 18
   acaagaattc gctaagagtg tcggcactcc 30
<210> 19
   <211> 30
   <212> DNA
   <213> Artificial Sequence
<220>
   <223> primer
<400> 19
   ggccaagctt ccgaagaaga caccatcaag 30
<210> 20
   <211> 6436
   <212> DNA
   <213> Artificial Sequence
<220>
   <223> vector
<400> 20
   ttcatgtcta accgtttgga tggtaaggta gcaatcatta caggtggtac gttgggtatc 60
   ggtttagcta tcgccacgaa gttcgttgaa gaaggggcta aggtcatgat taccggccgg 120
   cacagcgatg ttggtgaaaa agcagctaag agtgtcggca ctcctgatca gattcaattt 180
   ttccaacatg attcttccga tgaagacggc tggacgaaat tattcgatgc aacggaaaaa 240
   gcctttggcc cagtttctac attagttaat aacgctggga tcatggttaa caagagtgtc 300
   gaagaaacca cgactgctga atggcgtaaa ttattagccg tcaaccttga tggtgtcttc 360
   ttcggtaccc gattagggat tcaacggatg aagaacaaag gcttaggggc ttccatcatc 420
   aacatgtctt cgatcgaagg ctttgtgggt gatcctagct taggggctta caacgcatct 480
   aaaggggccg tacggattat gtccaagtca gctgccttag attgtgccct aaaggactac 540
   gatgttcggg taaacactgt tcaccctggc tacatcaaga caccattggt tgatgaccta 600
   ccaggggccg aagaagcgat gtcacaacgg accaagacgc caatgggcca tatcggtgaa 660
   cctaacgata ttgcctacat ctgtgtttac ttggcttcta acgaatctaa atttgcaacg 720
   ggttctgaat ttgtagttga cggtggctac actgctcaat agtaagcttc tgttttggcg 780
   gatgagagaa gattttcagc ctgatacaga ttaaatcaga acgcagaagc ggtctgataa 840
   aacagaattt gcctggcggc agtagcgcgg tggtcccacc tgaccccatg ccgaactcag 900
   aagtgaaacg ccgtagcgcc gatggtagtg tggggtctcc ccatgcgaga gtagggaact 960
   gccaggcatc aaataaaacg aaaggctcag tcgaaagact gggcctttcg ttttatctgt 1020
   tgtttgtcgg tgaacgctct cctgagtagg acaaatccgc cgggagcgga tttgaacgtt 1080
   gcgaagcaac ggcccggagg gtggcgggca ggacgcccgc cataaactgc caggcatcaa 1140
   attaagcaga aggccatcct gacggatggc ctttttgcgt ttctacaaac tcttttgttt 1200
   atttttctaa atacattcaa atatgtatcc gctcatgaga caataaccct gataaatgct 1260
   tcaataatat tgaaaaagga agagtatgag tattcaacat ttccgtgtcg cccttattcc 1320
   cttttttgcg gcattttgcc ttcctgtttt tgctcaccca gaaacgctgg tgaaagtaaa 1380
   agatgctgaa gatcagttgg gtgcacgagt gggttacatc gaactggatc tcaacagcgg 1440
   taagatcctt gagagttttc gccccgaaga acgttttcca atgatgagca cttttaaagt 1500
   tctgctatgt ggcgcggtat tatcccgtgt tgacgccggg caagagcaac tcggtcgccg 1560
   catacactat tctcagaatg acttggttga gtactcacca gtcacagaaa agcatcttac 1620
   ggatggcatg acagtaagag aattatgcag tgctgccata accatgagtg ataacactgc 1680
   ggccaactta cttctgacaa cgatcggagg accgaaggag ctaaccgctt ttttgcacaa 1740
   catgggggat catgtaactc gccttgatcg ttgggaaccg gagctgaatg aagccatacc 1800
   aaacgacgag cgtgacacca cgatgcctgt agcaatggca acaacgttgc gcaaactatt 1860
   aactggcgaa ctacttactc tagcttcccg gcaacaatta atagactgga tggaggcgga 1920
   taaagttgca ggaccacttc tgcgctcggc ccttccggct ggctggttta ttgctgataa 1980
   atctggagcc ggtgagcgtg ggtctcgcgg tatcattgca gcactggggc cagatggtaa     2040
   gccctcccgt atcgtagtta tctacacgac ggggagtcag gcaactatgg atgaacgaaa     2100
   tagacagatc gctgagatag gtgcctcact gattaagcat tggtaactgt cagaccaagt     2160
   ttactcatat atactttaga ttgatttaaa acttcatttt taatttaaaa ggatctaggt     2220
   gaagatcctt tttgataatc tcatgaccaa aatcccttaa cgtgagtttt cgttccactg     2280
   agcgtcagac cccgtagaaa agatcaaagg atcttcttga gatccttttt ttctgcgcgt     2340
   aatctgctgc ttgcaaacaa aaaaaccacc gctaccagcg gtggtttgtt tgccggatca     2400
   agagctacca actctttttc cgaaggtaac tggcttcagc agagcgcaga taccaaatac     2460
   tgtccttcta gtgtagccgt agttaggcca ccacttcaag aactctgtag caccgcctac     2520
   atacctcgct ctgctaatcc tgttaccagt ggctgctgcc agtggcgata agtcgtgtct     2580
   taccgggttg gactcaagac gatagttacc ggataaggcg cagcggtcgg gctgaacggg     2640
   gggttcgtgc acacagccca gcttggagcg aacgacctac accgaactga gatacctaca     2700
   gcgtgagcta tgagaaagcg ccacgcttcc cgaagggaga aaggcggaca ggtatccggt     2760
   aagcggcagg gtcggaacag gagagcgcac gagggagctt ccagggggaa acgcctggta     2820
   tctttatagt cctgtcgggt ttcgccacct ctgacttgag cgtcgatttt tgtgatgctc     2880
   gtcagggggg cggagcctat ggaaaaacgc cagcaacgcg gcctttttac ggttcctggc     2940
   cttttgctgg ccttttgctc acatgttctt tcctgcgtta tcccctgatt ctgtggataa     3000
   ccgtattacc gcctttgagt gagctgatac cgctcgccgc agccgaacga ccgagcgcag     3060
   cgagtcagtg agcgaggaag cggaagagcg cctgatgcgg tattttctcc ttacgcatct     3120
   gtgcggtatt tcacaccgca tatggtgcac tctcagtaca atctgctctg atgccgcata     3180
   gttaagccag tatacactcc gctatcgcta cgtgactggg tcatggctgc gccccgacac     3240
   ccgccaacac ccgctgacgc gccctgacgg gcttgtctgc tcccggcatc cgcttacaga     3300
   caagctgtga ccgtctccgg gagctgcatg tgtcagaggt tttcaccgtc atcaccgaaa     3360
   cgcgcgaggc agctgcggta aagctcatca gcgtggtcgt gaagcgattc acagatgtct     3420
   gcctgttcat ccgcgtccag ctcgttgagt ttctccagaa gcgttaatgt ctggcttctg     3480
   ataaagcggg ccatgttaag ggcggttttt tcctgtttgg tcacttgatg cctccgtgta     3540
   agggggaatt tctgttcatg ggggtaatga taccgatgaa acgagagagg atgctcacga     3600
   tacgggttac tgatgatgaa catgcccggt tactggaacg ttgtgagggt aaacaactgg     3660
   cggtatggat gcggcgggac cagagaaaaa tcactcaggg tcaatgccag cgcttcgtta     3720
   atacagatgt aggtgttcca cagggtagcc agcagcatcc tgcgatgcag atccggaaca     3780
   taatggtgca gggcgctgac ttccgcgttt ccagacttta cgaaacacgg aaaccgaaga     3840
   ccattcatgt tgttgctcag gtcgcagacg ttttgcagca gcagtcgctt cacgttcgct 3900
   cgcgtatcgg tgattcattc tgctaaccag taaggcaacc ccgccagcct agccgggtcc 3960
   tcaacgacag gagcacgatc atgcgcaccc gtggccagga cccaacgctg cccgagatgc 4020
   gccgcgtgcg gctgctggag atggcggacg cgatggatat gttctgccaa gggttggttt 4080
   gcgcattcac agttctccgc aagaattgat tggctccaat tcttggagtg gtgaatccgt 4140
   tagcgaggtg ccgccggctt ccattcaggt cgaggtggcc cggctccatg caccgcgacg 4200
   caacgcgggg aggcagacaa ggtatagggc ggcgcctaca atccatgcca acccgttcca 4260
   tgtgctcgcc gaggcggcat aaatcgccgt gacgatcagc ggtccagtga tcgaagttag 4320
   gctggtaaga gccgcgagcg atccttgaag ctgtccctga tggtcgtcat ctacctgcct 4380
   ggacagcatg gcctgcaacg cgggcatccc gatgccgccg gaagcgagaa gaatcataat 4440
   ggggaaggcc atccagcctc gcgtcgcgaa cgccagcaag acgtagccca gcgcgtcggc 4500
   cgccatgccg gcgataatgg cctgcttctc gccgaaacgt ttggtggcgg gaccagtgac 4560
   gaaggcttga gcgagggcgt gcaagattcc gaataccgca agcgacaggc cgatcatcgt 4620
   cgcgctccag cgaaagcggt cctcgccgaa aatgacccag agcgctgccg gcacctgtcc 4680
   tacgagttgc atgataaaga agacagtcat aagtgcggcg acgatagtca tgccccgcgc 4740
   ccaccggaag gagctgactg ggttgaaggc tctcaagggc atcggtcgac caaactaaag 4800
   cgcccttgtg gcgctttagt tttgttcatc ttccagcaag cgtgcgccgg taccttcttc 4860
   tcctaagcgg tcgcccgggt tacgcaacgg gcaatcactg cgcgaaaggc agccacaacc 4920
   aatacatccg tccagttcgt cacgcagcgc cactaaggta tgaatgcgcc gatccaactc 4980
   ttctcgccat tgggacgaaa gctgtttcca ctctttcgca cttaacgtat gcccttcggg 5040
   caacacgcca aacgcttcac caatggtcgc cagcggaatg ccaatacgct gagcaatttt 5100
   gataattgca acatatcgca acacatcacg tttatatcgc cgctgattgc cgctgttacg 5160
   gatactggta atcaaccctt tactttcata gaaatgcagc gccgataccg ccacaccgct 5220
   gcgtttcgcc acttcgccgg gggttagcag cgctttaatg cggggtaatt tcttttccat 5280
   aaatcgcttt acctcaagtt aacttgagga attatactcc ccaacagatg aattaacgaa 5340
   ctgaacactg aaaagaggca gatttatgtc ccatcagaaa attattcagg atcttatcgc 5400
   atggattgac gagcatattg accagccgta agcatgcaag gagaattaca tggtgagcaa 5460
   gggcgaggag ctgttcaccg ggtggtgccc catcctggtc gagctggacg gcgacgtaaa 5520
   cggccacaag ttcagcgtgt ccggcgaggg cgagggcgat gccacctacg gcaagctgac 5580
   cctgaagttc atctgcacca ccggcaagct gcccgtgccc tggcccaccc tcgtgaccac 5640
   cttcggctac ggcctgcagt gcttcgcccg ctaccccgac cacatgaagc agcacgactt 5700
   cttcaagtcc gccatgcccg aaggctacgt ccaggagcgc accatcttct tcaaggacga 5760
   cggcaactac aagacccgcg ccgaggtgaa gttcgagggc gacaccctgg tgaaccgcat 5820
   cgagctgaag ggcatcaact tcaaggagga cggcaacatc ctggggcaca agctggagta 5880
   caactacaac agccacaacg tctatatcat ggccgacaag cagaagaacg gcatcaaggt 5940
   gaacttcaag atccgccaca acatcgaggg cggcagcgtg cagctcgccg accactacca 6000
   gcagaacacc cccatcggcg acggccccgt gctgctgccc gacaaccact acctgagcta 6060
   ccagtccgcc ctgagcaaag accccaacga gaagcgcgat cacatggtcc tgctggagtt 6120
   cgtgaccgcc gccgggatca ctctcggcat ggacgagctg tacaagtaat aaatcgatcc 6180
   ggagcttatc gactgcacgg tgcaccaatg cttctggcgt caggcagcca tcggaagctg 6240
   tggtatggct gtgcaggtcg taaatcactg cataattcgt gtcgctcaag gcgcactccc 6300
   gttctggata atgttttttg cgccgacatc ataacggttc tggcaaatat tctgaaatga 6360
   gctgttgaca attaatcatc ggctcgtata atgtgtggaa ttgtgagcgg ataacaattt 6420
   cacacaggaa acagaa 6436
<210> 21
   <211> 31
   <212> DNA
   <213> Artificial Sequence
<220>
   <223> SoxS_for_SphI_t1
<400> 21
   atctgcatgc cggctggtca atatgctcgt c 31
<210> 22
   <211> 30
   <212> DNA
   <213> Artificial Sequence
<220>
   <223> SoxR_rev_SalI_t1
<400> 22
   gctagtcgac caaactaaag cgcccttgtg 30
<210> 23
   <211> 26
   <212> DNA
   <213> Artificial Sequence
<220>
   <223> EYFP_for_SphI_t1
<400> 23
   agaggcatgc gtgagcaagg gcgagg 26
<210> 24
   <211> 35
   <212> DNA
   <213> Artificial Sequence
<220>
   <223> EYFP_rev_ClaI_t1
<400> 24
   gcgcatcgat ttattacttg tacagctcgt ccatg 35
<210> 25
   <211> 6418
   <212> DNA
   <213> Artificial Sequence
<220>
   <223> pSenSox_t1
<400> 25
   ttcatgtcta accgtttgga tggtaaggta gcaatcatta caggtggtac gttgggtatc      60
   ggtttagcta tcgccacgaa gttcgttgaa gaaggggcta aggtcatgat taccggccgg     120
   cacagcgatg ttggtgaaaa agcagctaag agtgtcggca ctcctgatca gattcaattt     180
   ttccaacatg attcttccga tgaagacggc tggacgaaat tattcgatgc aacggaaaaa     240
   gcctttggcc cagtttctac attagttaat aacgctggga tcgcggttaa caagagtgtc     300
   gaagaaacca cgactgctga atggcgtaaa ttattagccg tcaaccttga tggtgtcttc     360
   ttcggtaccc gattagggat tcaacggatg aagaacaaag gcttaggggc ttccatcatc     420
   aacatgtctt cgatcgaagg ctttgtgggt gatcctagct taggggctta caacgcatct     480
   aaaggggccg tacggattat gtccaagtca gctgccttag attgtgccct aaaggactac     540
   gatgttcggg taaacactgt tcaccctggc tacatcaaga caccattggt tgatgaccta     600
   ccaggggccg aagaagcgat gtcacaacgg accaagacgc caatgggcca tatcggtgaa     660
   cctaacgata ttgcctacat ctgtgtttac ttggcttcta acgaatctaa atttgcaacg     720
   ggttctgaat ttgtagttga cggtggctac actgctcaat agtaagcttc tgttttggcg     780
   gatgagagaa gattttcagc ctgatacaga ttaaatcaga acgcagaagc ggtctgataa     840
   aacagaattt gcctggcggc agtagcgcgg tggtcccacc tgaccccatg ccgaactcag     900
   aagtgaaacg ccgtagcgcc gatggtagtg tggggtctcc ccatgcgaga gtagggaact     960
   gccaggcatc aaataaaacg aaaggctcag tcgaaagact gggcctttcg ttttatctgt    1020
   tgtttgtcgg tgaacgctct cctgagtagg acaaatccgc cgggagcgga tttgaacgtt    1080
   gcgaagcaac ggcccggagg gtggcgggca ggacgcccgc cataaactgc caggcatcaa    1140
   attaagcaga aggccatcct gacggatggc ctttttgcgt ttctacaaac tcttttgttt    1200
   atttttctaa atacattcaa atatgtatcc gctcatgaga caataaccct gataaatgct    1260
   tcaataatat tgaaaaagga agagtatgag tattcaacat ttccgtgtcg cccttattcc    1320
   cttttttgcg gcattttgcc ttcctgtttt tgctcaccca gaaacgctgg tgaaagtaaa 1380
   agatgctgaa gatcagttgg gtgcacgagt gggttacatc gaactggatc tcaacagcgg 1440
   taagatcctt gagagttttc gccccgaaga acgttttcca atgatgagca cttttaaagt 1500
   tctgctatgt ggcgcggtat tatcccgtgt tgacgccggg caagagcaac tcggtcgccg 1560
   catacactat tctcagaatg acttggttga gtactcacca gtcacagaaa agcatcttac 1620
   ggatggcatg acagtaagag aattatgcag tgctgccata accatgagtg ataacactgc 1680
   ggccaactta cttctgacaa cgatcggagg accgaaggag ctaaccgctt ttttgcacaa 1740
   catgggggat catgtaactc gccttgatcg ttgggaaccg gagctgaatg aagccatacc 1800
   aaacgacgag cgtgacacca cgatgcctgt agcaatggca acaacgttgc gcaaactatt 1860
   aactggcgaa ctacttactc tagcttcccg gcaacaatta atagactgga tggaggcgga 1920
   taaagttgca ggaccacttc tgcgctcggc ccttccggct ggctggttta ttgctgataa 1980
   atctggagcc ggtgagcgtg ggtctcgcgg tatcattgca gcactggggc cagatggtaa 2040
   gccctcccgt atcgtagtta tctacacgac ggggagtcag gcaactatgg atgaacgaaa 2100
   tagacagatc gctgagatag gtgcctcact gattaagcat tggtaactgt cagaccaagt 2160
   ttactcatat atactttaga ttgatttaaa acttcatttt taatttaaaa ggatctaggt 2220
   gaagatcctt tttgataatc tcatgaccaa aatcccttaa cgtgagtttt cgttccactg 2280
   agcgtcagac cccgtagaaa agatcaaagg atcttcttga gatccttttt ttctgcgcgt 2340
   aatctgctgc ttgcaaacaa aaaaaccacc gctaccagcg gtggtttgtt tgccggatca 2400
   agagctacca actctttttc cgaaggtaac tggcttcagc agagcgcaga taccaaatac 2460
   tgtccttcta gtgtagccgt agttaggcca ccacttcaag aactctgtag caccgcctac 2520
   atacctcgct ctgctaatcc tgttaccagt ggctgctgcc agtggcgata agtcgtgtct 2580
   taccgggttg gactcaagac gatagttacc ggataaggcg cagcggtcgg gctgaacggg 2640
   gggttcgtgc acacagccca gcttggagcg aacgacctac accgaactga gatacctaca 2700
   gcgtgagcta tgagaaagcg ccacgcttcc cgaagggaga aaggcggaca ggtatccggt 2760
   aagcggcagg gtcggaacag gagagcgcac gagggagctt ccagggggaa acgcctggta 2820
   tctttatagt cctgtcgggt ttcgccacct ctgacttgag cgtcgatttt tgtgatgctc 2880
   gtcagggggg cggagcctat ggaaaaacgc cagcaacgcg gcctttttac ggttcctggc 2940
   cttttgctgg ccttttgctc acatgttctt tcctgcgtta tcccctgatt ctgtggataa 3000
   ccgtattacc gcctttgagt gagctgatac cgctcgccgc agccgaacga ccgagcgcag 3060
   cgagtcagtg agcgaggaag cggaagagcg cctgatgcgg tattttctcc ttacgcatct 3120
   gtgcggtatt tcacaccgca tatggtgcac tctcagtaca atctgctctg atgccgcata 3180
   gttaagccag tatacactcc gctatcgcta cgtgactggg tcatggctgc gccccgacac    3240
   ccgccaacac ccgctgacgc gccctgacgg gcttgtctgc tcccggcatc cgcttacaga    3300
   caagctgtga ccgtctccgg gagctgcatg tgtcagaggt tttcaccgtc atcaccgaaa    3360
   cgcgcgaggc agctgcggta aagctcatca gcgtggtcgt gaagcgattc acagatgtct    3420
   gcctgttcat ccgcgtccag ctcgttgagt ttctccagaa gcgttaatgt ctggcttctg    3480
   ataaagcggg ccatgttaag ggcggttttt tcctgtttgg tcacttgatg cctccgtgta    3540
   agggggaatt tctgttcatg ggggtaatga taccgatgaa acgagagagg atgctcacga    3600
   tacgggttac tgatgatgaa catgcccggt tactggaacg ttgtgagggt aaacaactgg    3660
   cggtatggat gcggcgggac cagagaaaaa tcactcaggg tcaatgccag cgcttcgtta    3720
   atacagatgt aggtgttcca cagggtagcc agcagcatcc tgcgatgcag atccggaaca    3780
   taatggtgca gggcgctgac ttccgcgttt ccagacttta cgaaacacgg aaaccgaaga    3840
   ccattcatgt tgttgctcag gtcgcagacg ttttgcagca gcagtcgctt cacgttcgct    3900
   cgcgtatcgg tgattcattc tgctaaccag taaggcaacc ccgccagcct agccgggtcc    3960
   tcaacgacag gagcacgatc atgcgcaccc gtggccagga cccaacgctg cccgagatgc    4020
   gccgcgtgcg gctgctggag atggcggacg cgatggatat gttctgccaa gggttggttt    4080
   gcgcattcac agttctccgc aagaattgat tggctccaat tcttggagtg gtgaatccgt    4140
   tagcgaggtg ccgccggctt ccattcaggt cgaggtggcc cggctccatg caccgcgacg    4200
   caacgcgggg aggcagacaa ggtatagggc ggcgcctaca atccatgcca acccgttcca    4260
   tgtgctcgcc gaggcggcat aaatcgccgt gacgatcagc ggtccagtga tcgaagttag    4320
   gctggtaaga gccgcgagcg atccttgaag ctgtccctga tggtcgtcat ctacctgcct    4380
   ggacagcatg gcctgcaacg cgggcatccc gatgccgccg gaagcgagaa gaatcataat    4440
   ggggaaggcc atccagcctc gcgtcgcgaa cgccagcaag acgtagccca gcgcgtcggc    4500
   cgccatgccg gcgataatgg cctgcttctc gccgaaacgt ttggtggcgg gaccagtgac    4560
   gaaggcttga gcgagggcgt gcaagattcc gaataccgca agcgacaggc cgatcatcgt    4620
   cgcgctccag cgaaagcggt cctcgccgaa aatgacccag agcgctgccg gcacctgtcc    4680
   tacgagttgc atgataaaga agacagtcat aagtgcggcg acgatagtca tgccccgcgc    4740
   ccaccggaag gagctgactg ggttgaaggc tctcaagggc atcggtcgac caaactaaag    4800
   cgcccttgtg gcgctttagt tttgttcatc ttccagcaag cgtgcgccgg taccttcttc    4860
   tcctaagcgg tcgcccgggt tacgcaacgg gcaatcactg cgcgaaaggc agccacaacc    4920
   aatacatccg tccagttcgt cacgcagcgc cactaaggta tgaatgcgcc gatccaactc    4980
   ttctcgccat tgggacgaaa gctgtttcca ctctttcgca cttaacgtat gcccttcggg    5040
   caacacgcca aacgcttcac caatggtcgc cagcggaatg ccaatacgct gagcaatttt    5100
   gataattgca acatatcgca acacatcacg tttatatcgc cgctgattgc cgctgttacg 5160
   gatactggta atcaaccctt tactttcata gaaatgcagc gccgataccg ccacaccgct 5220
   gcgtttcgcc acttcgccgg gggttagcag cgctttaatg cggggtaatt tcttttccat 5280
   aaatcgcttt acctcaagtt aacttgagga attatactcc ccaacagatg aattaacgaa 5340
   ctgaacactg aaaagaggca gatttatgtc ccatcagaaa attattcagg atcttatcgc 5400
   atggattgac gagcatattg accagccggc atgcgtgagc aagggcgagg agctgttcac 5460
   cggggtggtg cccatcctgg tcgagctgga cggcgacgta aacggccaca agttcagcgt 5520
   gtccggcgag ggcgagggcg atgccaccta cggcaagctg accctgaagt tcatctgcac 5580
   caccggcaag ctgcccgtgc cctggcccac cctcgtgacc accttcggct acggcctgca 5640
   gtgcttcgcc cgctaccccg accacatgaa gcagcacgac ttcttcaagt ccgccatgcc 5700
   cgaaggctac gtccaggagc gcaccatctt cttcaaggac gacggcaact acaagacccg 5760
   cgccgaggtg aagttcgagg gcgacaccct ggtgaaccgc atcgagctga agggcatcaa 5820
   cttcaaggag gacggcaaca tcctggggca caagctggag tacaactaca acagccacaa 5880
   cgtctatatc atggccgaca agcagaagaa cggcatcaag gtgaacttca agatccgcca 5940
   caacatcgag ggcggcagcg tgcagctcgc cgaccactac cagcagaaca cccccatcgg 6000
   cgacggcccc gtgctgctgc ccgacaacca ctacctgagc taccagtccg ccctgagcaa 6060
   agaccccaac gagaagcgcg atcacatggt cctgctggag ttcgtgaccg ccgccgggat 6120
   cactctcggc atggacgagc tgtacaagta ataaatcgat ccggagctta tcgactgcac 6180
   ggtgcaccaa tgcttctggc gtcaggcagc catcggaagc tgtggtatgg ctgtgcaggt 6240
   cgtaaatcac tgcataattc gtgtcgctca aggcgcactc ccgttctgga taatgttttt 6300
   tgcgccgaca tcataacggt tctggcaaat attctgaaat gagctgttga caattaatca 6360
   tcggctcgta taatgtgtgg aattgtgagc ggataacaat ttcacacagg aaacagaa 6418